# EUROPEAN PATENT SPECIFICATION

(11) **EP 4 619 105 B1**
(45) Date of publication and mention of the grant of the patent: **16.09.2026**
(21) Application number: 23821008.2
(22) Date of filing: 15.11.2023
(51) Int. Cl.: C07F 15/00, A61P 35/00, C09K 11/06, A61K 41/00

(54) **A RUTHENIUM (II) POLYPYRIDYL COMPLEX AS A PHOTO-SENSITISER IN PHOTODYNAMIC THERAPY, AND A PROCEDURE FOR MAKING SAID RUTHENIUM (II) POLYPYRIDYL COMPLEX**
RUTHENIUM(II)-POLYPYRIDYL-KOMPLEX ALS PHOTOSENSIBILISATOR IN DER PHOTODYNAMISCHEN THERAPIE UND VERFAHREN ZUR HERSTELLUNG DES RUTHENIUM(II)-POLYPYRIDYL-KOMPLEXES
COMPLEXE DE RUTHÉNIUM (II) POLYPYRIDYLE EN TANT QUE PHOTOSENSIBILISATEUR EN THÉRAPIE PHOTODYNAMIQUE ET PROCÉDÉ DE FABRICATION DUDIT COMPLEXE DE RUTHÉNIUM (II) POLYPYRIDYLE

(30) Priority: 18.11.2022 IT 202200023862
(43) Date of publication of application: 24.09.2025
(73) Proprietor: Università degli Studi di Firenze, 50121 Firenze (FI) (IT); Università Degli Studi di Cagliari, 09124 Cagliari (IT)
(72) Inventor: CASULA, Luca, 09039 Villacidro (SU) (IT); CONTI, Luca, 59100 Prato (PO) (IT); DIDDI, Alessia, 59100 Prato (IT); GIACOMAZZO, Gina Elena, 56023 Firenze (IT); GIORGI, Claudia, 50134 Firenze (IT); MURGIA, Sergio, 90125 Cagliari (IT); SCHLICH, Michele, 09126 Cagliari (IT); SINICO, Chiara, 09012 Capoterra (CA) (IT); VALTANCOLI, Barbara, 50019 Sesto Fiorentino (FI) (IT)
(74) Representative: ABM Agenzia Brevetti & Marchi
(86) International application number: PCT/IB2023/061573
(87) International publication number: WO 2024/105603

(56) References cited:
- WANG LI ET AL: "[pi]-Expansive Heteroleptic Ruthenium(II) Complexes as Reverse Saturable Absorbers and Photosensitizers for Photodynamic Therapy", INORGANIC CHEMISTRY, vol. 56, no. 6, 20 March 2017 (2017-03-20), Easton , US, pages 3245 - 3259, XP093041328, ISSN: 0020-1669, DOI: 10.1021/acs.inorgchem.6b02624
- DATABASE REAXYS [online] 1 January 2021 (2021-01-01), TOUPIN N: "Photosensitive Ru(II) Complexes as Inhibitors of the Major Human Drug Metabolizing Enzyme CYP3A4", XP093114361, Database accession no. XRN = 55416701
- DATABASE REAXYS [online] 1 January 2021 (2021-01-01), TOUPIN N: "Photosensitive Ru(II) Complexes as Inhibitors of the Major Human Drug Metabolizing Enzyme CYP3A4", XP093114362, Database accession no. XRN = 20011324
- TOUPIN NICHOLAS ET AL: "Photosensitive Ru(II) Complexes as Inhibitors of the Major Human Drug Metabolizing Enzyme CYP3A4", JOURNAL OF THE AMERICAN CHEMICAL SOCIETY, vol. 143, no. 24, 10 June 2021 (2021-06-10), pages 9191 - 9205, XP093041330, ISSN: 0002-7863, DOI: 10.1021/jacs.1c04155

## Description

### SCOPE OF THE INVENTION

The present invention relates to a Ru(II) polypyridyl complex for use as a photosensitiser, i.e. a photosensitising agent, in a photodynamic therapy for treating tumours, to a synthetic procedure thereto and to a medicament including such complex.

### PRIOR ART - TECHNICAL PROBLEMS

In recent years, photodynamic therapy has attracted increasing interest in the scientific community for the treatment of a wide variety of malignancies, as an alternative to chemotherapy and radiotherapy, in order to overcome well-known drawbacks, such as severe side effects, drug resistance and poor drug efficacy.

Photodynamic therapy involves the use of low-energy light-activated photosensitising agents that can be activated to produce highly cytotoxic reactive oxygen species (ROS), including singlet oxygen ¹O₂. Photodynamic therapy has the advantage of allowing a spatio-temporal control over the activation of the photosensitizer, permitting to improve the selectivity and efficacy of the treatment against selected tumoral targets, besides containing the side effects commonly associated with non-specific drug delivery.

A photosensitizer for photodynamic therapy must possess low toxicity under dark conditions and a high capacity to produce ROS after photoexcitation. Most to date known photosensitizers comprise tetrapyrrolic structures, such as porphyrins, phthalocyanines and chlorines. These drugs, despite their high capacity to produce cytotoxic species after irradiation, are poorly soluble in biological media, poorly selective, and induce prolonged photosensitivity in patients.

Some transition metal complexes, employed as photosensitising agents in photodynamic therapy, are likely to overcome these drawbacks. In particular, polypyridyl Ru(II) complexes show advantageous photophysical and electrochemical properties including high thermodynamic and kinetic stability, as well as the capability to generate singlet oxygen with high quantum yields. Examples of such compounds can be found in WO2020260424A1, EP3521295, CN113321687A, CN111875643A, CN110857310A, CN112266402A and WO2021032952A1.

In recent years, several Ru(II) polypyridyl complexes containing a benzo[i]dipyrido[3,2-a:2',3'-c]phenazine unit, hereafter referred to as 'dppn', have been synthesized. This ligand includes a particularly extensive aromatic system, which makes it possible to adjust the chemical-physical properties of the obtained compounds in a wide range, in particular, it allows to maximize the yield of singlet oxygen production, from which excellent therapeutic outcomes are derived.

A very limited number of polypyridyl complexes comprising two dppn units have also been synthesized. Two publications are known in this respect, which are mentioned below.

In a paper by Wang and co-workers¹, reference is made to a compound of formula:

[RuL(dppn)₂] ²⁺,

where L is 3,8-di(benzothiazolylfluorenyl)-1,10-phenanthroline, obtained by first introducing the two dppn units and then reacting the ligand L with the intermediate [Ru(dppn)₂Cl₂] in ethylene glycol. However, details on the way to carry out the synthesis of this intermediate are omitted and essential characterization data, such as ¹H NMR spectra of [RuL(dppn)₂]²⁺, were not provided. Such a synthetic route, which occurs *via* the intermediate [Ru(dppn)₂Cl₂], has the drawback that this intermediate is poorly soluble in most organic solvents, resulting in complications in the inherent synthesis.

A paper by Turro and co-workers² refers to the synthesis and characterization of a compound with formula:

[Ru(NN)(dppn)₂]²⁺,

where NN is 'bpy' = 2,2'-bipyridine. In this case, the preparation of the metal complex was carried out by the preliminary synthesis of the intermediate [Ru(NN)Cl₄] and then [Ru(NN)(CH₃CN)₄]²⁺, avoiding the use of [Ru(dppn)₂Cl₂], and introducing the two dppn groups only in the subsequent reaction steps. However, reaction times from 7 to 24 days were required to obtain [Ru(NN)Cl₄], which are very time-consuming and, therefore, industrially unacceptable. A number of complex purification steps are also required by this approach, leading to relatively low overall yields, of 28-37%. It is worthwhile to note that, in the above cited report by Turro, the compound [Ru(NN)(dppn)₂]²⁺ has only been studied as dye for solar cell applications (DSSC).

It is therefore desirable to define a route for the synthesis of polypyridyl Ru(II) complexes containing two dppn units that does not have the drawbacks of the prior art techniques briefly discussed above, i.e. a route providing intermediate compounds that are easily soluble in organic solvents and that allows the desired complexes to be obtained at higher yields and in shorter reaction times. Ru(II) complexes are also referred to in Toupin, N, Journal of the American Chemical Society, vol.143 No 24, pp 9191-9205 as inhibitors of the major human drug metabolizing enzyme CYP3A4, among which the complex of formula [Ru(dppz)₂(bpy)]²⁺ is mentioned.

### SUMMARY OF THE INVENTION

It is therefore an object of the present invention to provide a class of coordination compound or Ru(II) complexes that are suitable for the use as photosensitising agents in the photodynamic therapy of neoplasms.

It is another particular object of the invention to provide such a class of Ru(II) complexes that exhibit a greater selectivity towards tumour cells than the photosensitising agents for photodynamic therapy that are currently in use.

It is also a particular object of the invention to provide such a class of Ru(II) complexes that are capable of maximising the singlet oxygen production, thereby exhibiting better therapeutic efficiency than the photosensitising agents for photodynamic therapy that are currently in use.

It is another object of the present invention to provide an innovative and straightforward synthetic process for such a class of Ru(II) complexes that allows to obtain the above-mentioned complexes with higher yields and shorter reaction times, if compared to prior art procedures.

It is a further object of the invention to provide a medicament including such a Ru(II) complex, in which two dppn (benzo[i]dipyrido[3,2-a:2',3'-c]phenazine) ligands are present, while being adequately soluble in water and therefore in biological media, so as to improve absorption by a patient, and distribution to a target site in the patient's body without using potentially harmful co-solvents.

The above-mentioned objects are achieved by ruthenium (II) complexes or use as photosensitising agents in a photodynamic therapy for treating neoplasms as disclosed in claim 1, by a process for making said complexes as disclosed in claim 8, and by a photosensitizer medicament for photodynamic therapy as disclosed in claim 4. Advantageous embodiments of complexes and the medicament, as well as advantageous modification of the process, are defined in the respectively dependent claims.

According to one aspect of the invention, a class of ruthenium (II) coordination compounds having the following formula is described: 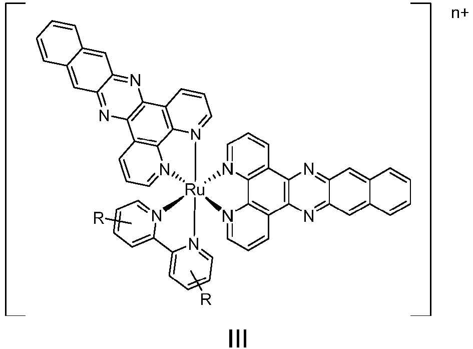
wherein n is selected between 0 and 2,
wherein R are substituent groups selected from the group consisting of:
   - a methyl group;
   - a phenyl group;
   - a -CH₂NH₂ group;
   - a -CH₂OH group;
   - a carboxyl group -COOH;
   - an ester group of formula -COOR₁, where R₁ can be selected from the group consisting of:
      - a linear or branched alkyl group with a number of carbon atoms in the range from 1 to 4;
      - an unsubstituted benzyl group;
      - a polyethylene glycol methoxy group with formula H(OCH₂CH₂)₁₋₄OH;
   - an amide group of formula -CONR₂R₃,
      where
      - R₂ can be hydrogen and R₃ can be a group selected from the group consisting of:
         - a linear or branched alkyl group with a number of carbon atoms in the range from 1 to 4;
         - a -(CH₂)₂NH₂ group;
         - a phenyl group,
         or
      - R₂ and R₃ can be linear or branched alkyl groups with a number of carbon atoms in the range from 1 to 4,
         or
      - NR₂R₃ can be a group selected from the group consisting of:
         - a cycloalkyl secondary amine with a number of carbon atoms in the range from 4 to 5;
         - a heterocycloalkyl secondary amine comprising a heteroatom selected between nitrogen and oxygen,
      for use as a photosensitising agents in the photodynamic therapy of neoplasms.

In other words, the invention relates to a plurality of polypyridyl complexes of ruthenium (II), hereinafter also referred to as [Ru(NN)(dppn)₂] ⁿ⁺, where
- dppn are benzo[i]di-pyrido[3,2-a:2',3'-c]phenazine units, i.e. bidentate heterocyclic ligands characterized by extensive aromaticity, and
- NN is a functionalised 2,2'-bipyridine ligand, whose R groups are described above.

The presence of the two dppn ligands in the same molecule makes it possible to maximize singlet oxygen production, therefore the above ruthenium (II) compounds turn out to be useful as photosensitising agents in a photodynamic therapy. More in detail, dppn ligands exhibit extensive π-conjugation, which allows them to easily interact with important biological targets such as proteins and/or DNA, in the latter case the interactions are π-stacking interactions with the DNA double helix. Moreover, the extensive π-conjugation of dppn prolongs the duration of the lowest-energy triplet excited states of the photosensitising agents, leading to improved production of singlet oxygen ¹O₂ due to excitation with appropriate low-energy light radiation, a key feature for the effectiveness of PS potentials.

In fact, as shown hereinafter, the obtained Ru(II) compounds exhibit excellent singlet oxygen production properties *via* photoexcitation, effectively interact with DNA as a possible biological target, and show remarkable phototoxicity against various cancer cell models.

According to another aspect of the invention, a process for obtaining a compound of a class of ruthenium (II) coordination compounds having the following formula: 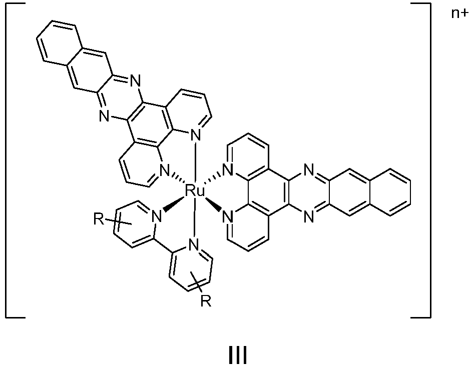
wherein n is selected between 0 and 2,
wherein R are substituent groups selected from the group consisting of:
   - hydrogen;
   - a methyl group,
   - a phenyl group,
   - a -CH₂NH₂ group,
   - a -CH₂OH group
   - a carboxyl group -COOH,
   - an ester group of formula -COOR₁, where R₁ can be selected from the group consisting of:
      - a linear or branched alkyl group with a number of carbon atoms in the range from 1 to 4;
      - an unsubstituted benzyl group;
      - a polyethylene glycol methoxy group with formula H(OCH₂CH₂)₁₋₄OH.
   - an amide group of formula -CONR₂R₃,
      where
      - R₂ can be hydrogen and R₃ can be a group selected from the group consisting of:
         - a linear or branched alkyl group with a number of carbon atoms in the range from 1 to 4;
         - a -(CH₂)₂NH₂ group;
         - a phenyl group,
         or
      - R₂ and R₃ can be linear or branched alkyl groups with a number of carbon atoms in the range from 1 to 4.
         or
      - NR₂R₃ can be a group selected from the group consisting of:
         - a cycloalkyl secondary amine with a number of carbon atoms in the range from 4 to 5;
         - a heterocycloalkyl secondary amine comprising a heteroatom selected between nitrogen and oxygen,
comprises the consecutive steps of:
- obtaining a reactive polymeric precursor [Ru(CO)₂Cl₂]ₘ by reaction between ruthenium (III) chloride and paraformaldehyde;
- obtaining a *trans*-Cl[Ru(NN)Cl₂(CO)₂] intermediate complex: 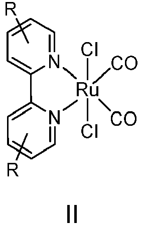
   where NN is a functionalised 2,2'-bipyridine ligand, whose R groups are described above,
   by reaction of the reactive polymer precursor [Ru(CO)₂Cl₂]ₘ with a predetermined symmetrically functionalised 2,2'-bipyridine ligand with two equal R functional groups;
- obtaining the ruthenium (II) coordination compound by reaction of the intermediate complex 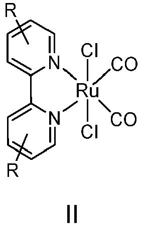 with benzo[i]dipyridine[3,2-a:2',3'-c]phenazine.

In other words, the present invention provides a straightforward synthetic route to heteroleptic ruthenium (II) complexes that include two extended aromatic bidentate ligands containing nitrogen atoms consisting in the benzo[i]dipyridine[3,2-a:2',3'-c]phenazine (dppn) units plus a symmetrically functionalised 2,2'-bipyridine ligand whose R-groups are indicated hereinafter, according to the general scheme below,
where complexes of formula III are represented without their charge, and in which three reaction steps are involved, i.e.: 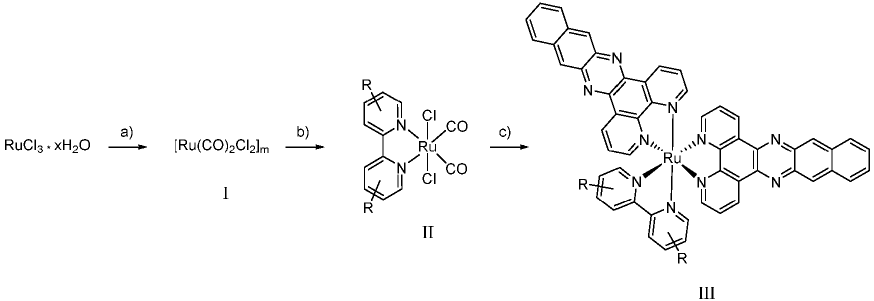
a) preparation of the polymeric precursor I;
b) preparation of *trans*-Cl[Ru(NN)Cl₂(CO)₂] complexes of formula II;
c) preparation of complexes of formula [Ru(NN)(dppn)₂]ⁿ⁺, with n = 0,2 (III) by reaction with two dppn equivalents.

This three-step synthetic procedure allows the desired compounds to be obtained with relatively high yields, normally between 22% and 47% on the overall reaction yields, and the reaction times are as long as a few hours at most.

In the process according to the invention, the NN ligands is introduced before the Ru(II)-coordination by the dppn ligands. In the present synthetic process, complexes of formula III are obtained through the preliminary synthesis of intermediates II, overcoming the problems associated to the scarce solubility in most organic solvents of the intermediate [Ru(dppn)₂Cl₂], conventionally employed in the synthesis of bis-heteroleptic Ru(II) polypyridyl complexes, see the above referenced document by Wang, cit.. Typically, this is also the strategy commonly employed to prepare Ru(II) complexes including only one dppn unit.

On the other hand, the process of the present invention allows obtaining the Ru(II) complexes employing a reaction time far shorter than the time required by the route according to Turro et al. (cit.)

The present invention provides a simple method for preparing a complex of the class of complexes [Ru(NN)(dppn)₂]ⁿ⁺ (n = 0, 2) with relatively high yields, relying on the preliminary preparation of the reactive polymeric compound [Ru(CO)₂Cl₂]ₘ (I), which is first reacted with the functionalized 2,2'-bipyridine unit to give the monomer *trans*-Cl[Ru(NN)Cl₂(CO)₂] (II), which is then allowed to react with two equivalents of dppn units to give compounds III. This approach makes it possible to overcome the solubility issues arising from the use of the intermediate [Ru(dppn)₂Cl₂], and allows obtaining variously substituted III compounds with good yields.

Moreover, with the synthetic route described here, reaction intermediates I and II can be used without requiring purification, which shortens the overall process times, reduces the required solvent amount and increases the yield. Only compounds III require purification before they can be handled for characterisation and further use.

The R-functions of the remaining bidentate NN ligands can be optimally selected to finely tune the chemical-physical properties of the obtained Ru(II) complexes.

Preferably, the 2,2'-bipyridine ligands are functionalized in the 5,5' and 4,4' positions with R substituents. In other words, each of said R substituent groups is bonded to a carbon atom at a position selected between the 5,5' position and the 4,4' position of each pyridine ring of the 2,2'-bipyridine group 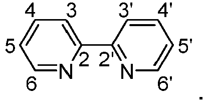 The positions indicated above are in fact those that make it possible to obtain compounds that are stable upon irradiation as they are free from such unfavourable conditions as steric encumbrance, and that, at the same time, do not entail complications when synthesising the complex.

In particular, the 2,2'-bipyridine ligand is symmetrically modified in the 5,5' or 4,4' positions with two R-substituents. In other words, the substituent groups R are bonded to carbon atoms at symmetrical 5,5' or 4,4' positions of respective pyridine rings of this 2,2'-bipyridine group. This makes it possible to obtain products as enantiomeric mixtures avoiding the formation of constitutional isomers.

According to another aspect of the invention, a photosensitising medicament for use in a photodynamic therapy for treating neoplasms is also provided, said medicament containing, as a photosensitising agent, a ruthenium (II) coordination compound having the formula: 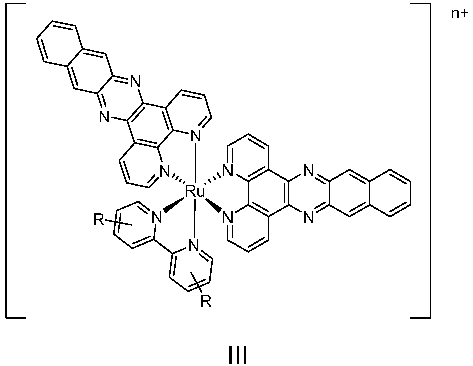
wherein n is selected between 0 and 2,
wherein R are substituent groups selected from the group consisting of:
   - hydrogen,
   - a methyl group,
   - a phenyl group,
   - a -CH₂NH₂ group,
   - a -CH₂OH group
   - a carboxyl group -COOH,
   - an ester group of formula -COOR₁, where R₁ can be selected from the group consisting of:
      - a linear or branched alkyl group with a number of carbon atoms in the range from 1 to 4;
      - an unsubstituted benzyl group;
      - a polyethylene glycol methoxy group with formula H(OCH₂CH₂)₁₋₄OH.
   - an amide group of formula -CONR₂R₃,
      where
      - R₂ can be hydrogen and R₃ can be a group selected from the group consisting of:
         - a linear or branched alkyl group with a number of carbon atoms in the range from 1 to 4;
         - a -(CH₂) ₂NH₂ group;
         - a phenyl group,
         or
      - R₂ and R₃ can be linear or branched alkyl groups with a number of carbon atoms in the range from 1 to 4.
         or
      - NR₂R₃ can be a group selected from the group consisting of:
         - a cycloalkyl secondary amine with a number of carbon atoms in the range from 4 to 5;
         - a heterocycloalkyl secondary amine comprising a heteroatom selected between nitrogen and oxygen.
      wherein the coordination compound is incorporated into nanoparticles, in particular, it is incorporated in liquid-crystalline lamellar and/or non-lamellar liquid-crystalline lipid nanoparticles. The effect of such incorporation consists in improving water solubility of the coordination compound and, therefore, in allowing its administration without using any co-solvent.

In other words, the invention relates to a plurality of polypyridyl ruthenium (II) complexes, hereinafter also referred to as [Ru(NN)(dppn)₂]ⁿ⁺, wherein, in each of said complexes,
- dppn are benzo[i]di-pyrido[3,2-a:2',3'-c]phenazine units, i.e. bidentate heterocyclic ligands characterised by extensive aromaticity, and
- NN is a functionalized 2,2'-bipyridine ligand, wherein the R groups are as described above.

This allows for one or more therapeutic advantages among:
a) more uniform and/or more selective medicament distribution at the tumour site;
b) crossing epithelial and organ barriers, depending on the route of administration. For example, such barriers may be the stratum corneum of the skin, blood-brain barrier, oesophageal or intestinal mucosa and others;
c) modulation of release and half-life in circulation;
d) reduction of toxicity at non-target sites.

Preferably, the liquid-crystalline lipid nanoparticles are cubosomes.

Cubosomes, often referred to as "bicontinuous cubic liquid crystalline nanoparticles", are peculiar nanoparticles used for the delivery of lipophilic and/or hydrophilic drugs. They are characterized by a three-dimensional, honeycomb-like arrangement of a lipid bilayer, with inner aqueous channels, and can encapsulate numerous bioactive components, such as pharmacological agents, proteins and amino acids. Generally, cubosomes exhibit several benefits over other nanoparticles, in particular, liposomes, taken as golden standard in nanomedicine, in terms of:
- stability: from a colloidal perspective, cubosomes exhibit a greater breaking resistance, a quality that is critical for drug delivery as it increases the probability that the medication will reach the target location without deteriorating or losing its effectiveness;
- increased drug loading: cubosomes exhibit a larger bilayer area to particle volume ratio, and they show a huge internal surface area that allows carrying of large pharmacological payloads;
- improved bioavailability: by overcoming solubility and stability drawbacks, cubosomes can increase drugs bioavailability, which allows a lower drug dose, thus reducing the possibility of adverse effects.

As a result of the above, cubosomes have shown a higher encapsulation efficiency and a more potent pharmacological effect, both in vitro and in vivo, in comparison e.g. with liposomes.

Similarly, cubosomes showed higher in vitro cytotoxicity, cellular uptake and tumor growth inhibition when compared to solid lipid nanoparticles (SLNs) loaded with a same drug. Moreover, when applied on the skin, cubosomes have shown higher drug penetration compared to liposomes, transfer-somes, and ethosomes.

As an alternative, the liquid-crystalline lipid nanoparticles can be selected from the group consisting of:
- hexosomes;
- solid lipid nanoparticles;
- nanoemulsions;
- a combination of the above.
As a further alternative, the liquid-crystalline lipid nanoparticles could also be liposomes.

The references to methods of treatment in the subsequent paragraphs of this description are to be interpreted as references to the compounds, pharmaceutical compositions and medicaments of the present invention for use in a method for treatment of the human body by therapy (or for diagnosis where appropriate). Disclosed is a photodynamic therapeutic method for treating neoplasms comprising a step of administering to a patient a medicament containing the nanoformulated coordination compound described above as a photosensitising agent, and a step of administering light to a neoplastic area in order to trigger the singlet oxygen production and induce site-specific cytotoxicity.

### BRIEF DESCRIPTION OF THE DRAWINGS

Further features and advantages of the present invention as defined in the claims will be better understood by the following description of variants and embodiments thereof, made by way of example and not of limitation, with reference to the attached figures, in which
- Fig. 1 shows the ¹H-NMR spectrum of complex Illa in (CD₃)₂CO);
- Fig. 2 shows the ¹H ¹H-COSY spectrum of complex IIIa in (CD₃)₂CO);
- Fig. 3 shows the ¹³C-NMR spectrum of complex Illa in (CD₃)₂CO;
- Fig. 4 shows the HSQC spectrum of complex Illa in (CD₃)₂CO);
- Fig. 5 shows the HR MS spectrum of complex Illa;
- Fig. 6 shows the ¹H-NMR spectrum of complex IIIb in (CD₃)₂SO;
- Fig. 7 shows the ¹H¹H-COSY spectrum of complex IIIb in (CD₃)₂SO;
- Fig. 8 shows the HR MS spectrum of complex IIIb;
- Fig. 9 shows the ¹H-NMR spectrum of complex IIIc in (CD₃)₂CO;
- Fig. 10 shows the ¹H¹H-COSY spectrum of complex IIIc in (CD₃)₂CO;
- Fig. 11 shows the ¹³C NMR spectrum of complex IIIc in (CD₃)₂CO;
- Fig. 12 shows the HSQC spectrum of complex IIIc in (CD₃)₂CO;
- Fig. 13 shows the HR MS spectrum of complex IIIc;
- Fig. 14 shows the absorption spectra of complexes IIIa, IIIb and IIIc in CH₃CN;
- Fig. 15 shows the absorption spectra of a solution containing DHN (330 µM) and the complexes IIIa, IIIb and IIIc (10 µM) in acetonitrile for different irradiation times, up to 200 seconds, with a 30W LED lamp, and an emission spectrum width ranging from 400 nm to 500 nm;
- Fig. 16 shows the semilogarithmic graphs of In(Aₜ/Aₒ) as a function of an irradiation time for the complexes IIIa, IIIb and IIIc and for [Ru(phen)₃]Cl₂, the latter taken as a reference ([DHN]= 330 µM, [Ru]= 10 µM);
- Fig. 17 shows the absorption spectra of buffered Tris-HCl 10 mM, NaCl 50 mM solutions at pH 7.2 of complexes Illa, IIIb and IIIc (10 µM), said spectra detected in the presence of increasing concentrations of double-stranded DNA;
- Fig. 18 shows the graphs of the [DNA]/|εₐ-ε_{b} | values against the molar concentration of DNA for the complexes Illa, IIIb and IIIc, Ru=10 µM, Tris-HCl buffer 10 mM, NaCl 50 mM, pH 7.2;
- Fig. 19 shows the results of cytotoxicity tests of complex IIIa, at different concentrations thereof, on squamous cell carcinoma of the epidermis (A431) and glioblastoma (U87MG) models, after irradiation with LED light for 30 minutes and in the dark;
- Fig. 20 shows the results of cytotoxicity tests of complex IIIb, at different concentrations thereof, on the squamous cell carcinoma model of the epidermis (A431), after irradiation with LED light for 30 minutes and in the dark;
- Fig. 21 shows the results of cytotoxicity tests of complex IIIb encapsulated in cubosomes, at different concentrations, on the squamous cell carcinoma model of the epidermis (A431) after irradiation with LED light for 30 minutes and in the dark;
- Fig. 22 shows the results of cytotoxicity tests of complex IIIc, at different concentrations thereof, on the Methastatic Lung Adenocarcinoma (Calu-3), after irradiation with LED light for 30 minutes and in the dark;
- Fig. 23 shows the results of cytotoxicity tests of complex IIIc encapsulated in cubosomes, at different concentrations, on the Methastatic Lung Adenocarcinoma (Calu-3) after irradiation with LED light for 30 minutes and in the dark;
- Fig. 24 shows a transmission electron microscopy image at cryogenic temperatures of cubosomes loaded with the complex IIIb;
- Fig. 25 shows a SAXS diffractogram of cubosomes loaded with complex IIIb, in which the Bragg peaks corresponding to the bicontinuous cubic phases Im3m and Pn3m are indicated by the dashed and continuous vertical lines, respectively;
- Fig. 26 shows the results of stability tests of the cubosome formulation.

### EXAMPLES

Three examples of Ru(II) complexes containing two benzo[i]dipyrido[3,2-a:2',3'-c]phenazine (dppn) units, are described hereinafter. These exemplary complexes can be represented by the common formula: 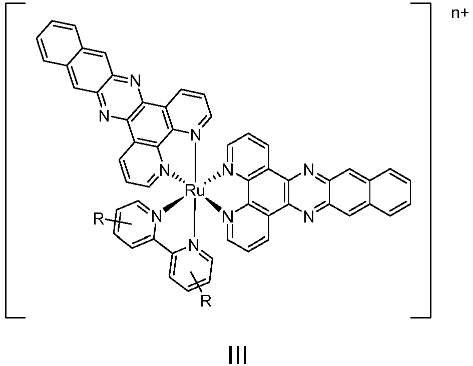 and are denoted Illa, IIIb and IIIc, according to whether R is a methyl group, a carboxylate group and a morpholinomethanone group, respectively. Therefore, complexes IIIa, IIIb and IIIc have the formulae shown below, in which the charge n+ is omitted:

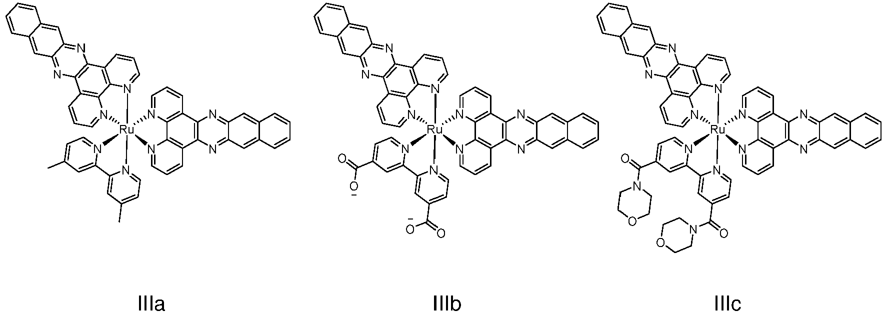

A synthetic route to obtain the above complexes, according to the process of the present invention, is diagrammatically shown below, including steps a), b) and c) leading to intermediates I and II, and to the desired Ru(II) complexes (III), respectively: 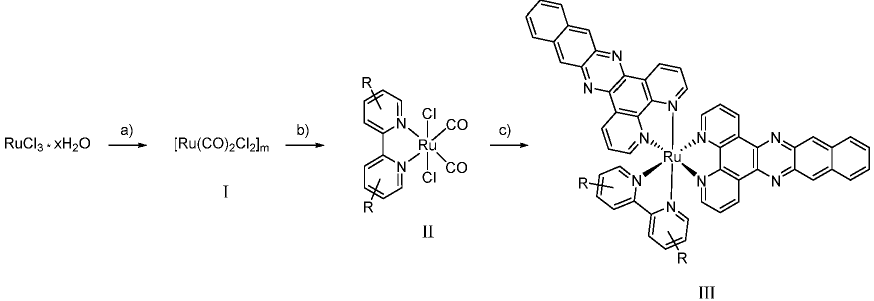 Even in this case, the charge of complex III is omitted for the sake of simplicity.

### Synthesis

### Step (a): preparation of the polymer precursor 'I'

This common intermediate was prepared in all three cases by refluxing commercial ruthenium trichloride, RuCl₃·xH₂O, and paraformaldehyde in a 1.4:1 w/w in 90% formic acid during 6 hours. The resulting polymer I was isolated as a pale-yellow powder through trituration from hexane. The polymer I precursor was obtained at an 80% yield.

### (b) preparation of the trans-Cl[Ru(NN)Cl₂(CO)₂] intermediates, 'II'

The NN ligands corresponding to complexes IIIa, IIIb and IIIc were inserted into the coordination sphere of the Ru(II) centres, leading to the respective intermediate complexes II *trans*-Cl[Ru(NN)Cl₂(CO)₂]. The polymer precursor I was reacted with the NN ligands in a 1:1 molar ratio in hot methanol or dimethylformamide, depending on the solubility of the specific NN ligand employed. Intermediate complexes II were obtained by a simple hot filtration of the reaction mixture, with high yields, ranging between 55% and 75%.

### (c) preparation of the complexes 'IIIa-IIIc' from corresponding intermediates II

In the third step of the synthetic route to complexes Illa, IIIb and IIIc, two equivalents of dppn are allowed to react in each case with the respective precursor II in refluxed 2-methoxyethanol as a solvent in the presence of 5 equivalents of trimethylamine N-oxide, leading to the formation of the respective complexes III of formula [Ru(NN)(dppn)₂]ⁿ⁺ (n = 0, 2). The complexes are then precipitated by adding a 0.1 M solution of KPF₆, filtered under vacuum and collected with a high degree of purity. When necessary, the complexes are subsequently purified by flash chromatography on silica gel, using a dichloromethane and methanol gradient mixture as the eluent. The desired products are therefore obtained as hexafluorophosphate salts [Ru(NN)(dppn)₂](PF₆)₂ with yields ranging between 50% and 78%.

A detailed description of step (c) of the synthesis of each complex IIIa, IIIb and IIIc is given hereinafter.

### [Ru(4,4'-dimethyl-2,2-bipyridine)(benzo[i]dipyrido[3,2-a:2',3'-c]phenazine)₂][PF₆]₂ (IIIa).

To a solution of *trans*-Cl[Ru(4,4'-dimethyl-2,2'-bipyridine)Cl₂(CO)₂] (80 mg, 0.19 mmol) in 8 mL of degassed 2-methoxyethanol were added benzo[i]dipyrido [3,2-a:2',3'-c]phenazine (dppn) (129 mg, 0.39 mmol) and trimethylamine N-oxide (106 mg, 0.95 mmol). The reaction mixture was stirred for 4 h at reflux under nitrogen atmosphere. After cooling to room temperature, the addition of 3 mL of 0.1 M KPF₆ solution allowed the complete precipitation of Illa. The precipitate was filtered and washed with water and chloroform. The crude product was purified by flash chromatography on silica gel (eluent: starting from DCM:MeOH 50:1 with 10% Acetone to DCM:MeOH 30:1 with 10% Acetone) to obtain Illa as a red powder at a 78% yield.

### [Ru(4,4'-dicarboxylate-2,2'-bipyridine)(benzo[i]dipyrido[3,2-a :2',3'-c]phenazine)₂] (IIIb)

To a solution of *trans*-Cl[Ru(4,4'-dicarboxylate-2,2'-bipyridine)Cl₂(CO)₂] (80 mg, 0.17 mmol) in 8 mL of degassed 2-methoxyethanol were added benzo[i]dipyrido[3,2-a:2',3'-c]phenazine (dppn) (113 mg, 0.34 mmol) and trimethylamine N-oxide (95 mg, 0.85 mmol). The reaction mixture was stirred during 6 hours at reflux under nitrogen atmosphere. After cooling to room temperature, the crude product was filtered and washed with water and then triturated with CHCl₃ to obtain (IIIb) as a red powder with a 50% yield.

### [Ru(4,4'-(diylbis(morpholinomethanone)-2,2'-bipyridine)(benzo[i]dipyrido[3,2-a :2',3'-c]phenazine)₂][PF₆]₂ (IIIc)

To a solution of *trans*-*Cl*[Ru(4,4'-(diylbis(morpholinomethanone)-2,2'-bipyridine)Cl₂(CO)₂] (100 mg, 0.16 mmol) in 8 mL of degassed 2-methoxyethanol was added benzo[i]dipyrido[3,2-a:2',3'-c]phenazine (dppn) (106 mg, 0.32 mmol) and trimethylamine N-oxide (116 mg, 1.04 mmol). The reaction mixture was stirred during 4 h at reflux under N atmosphere₂. After cooling to room temperature, the addition of 2 mL of 0.1 M aqueous solution of KPF₆ allowed the complete precipitation of complex IIIc. The crude product was filtered and washed with water, methanol and diethyl ether. Subsequently, it was purified by flash chromatography on silica gel (eluent: starting with DCM:MeOH 30:1 with 10% acetone) to obtain complex IIIc as a red solid with a yield of 62%.

The three-step synthesis process resulted in final products with yields ranging from 22% to 47%. The products were obtained pure and characterized by nuclear magnetic resonance ¹H, ¹H¹H COSY, ¹³C NMR and HR MS.

### Characterization of synthesized complexes: NMR

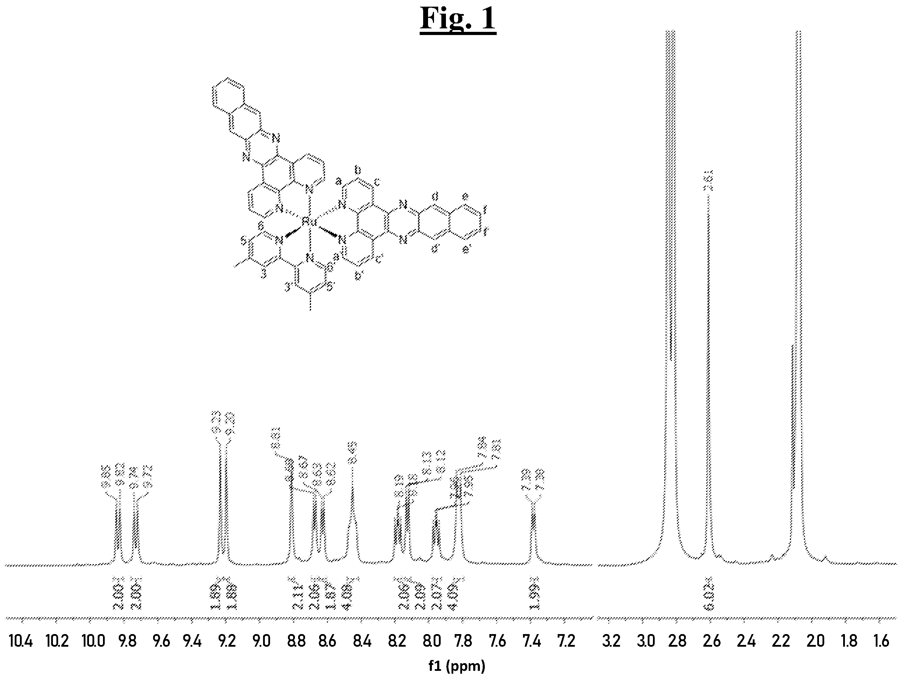

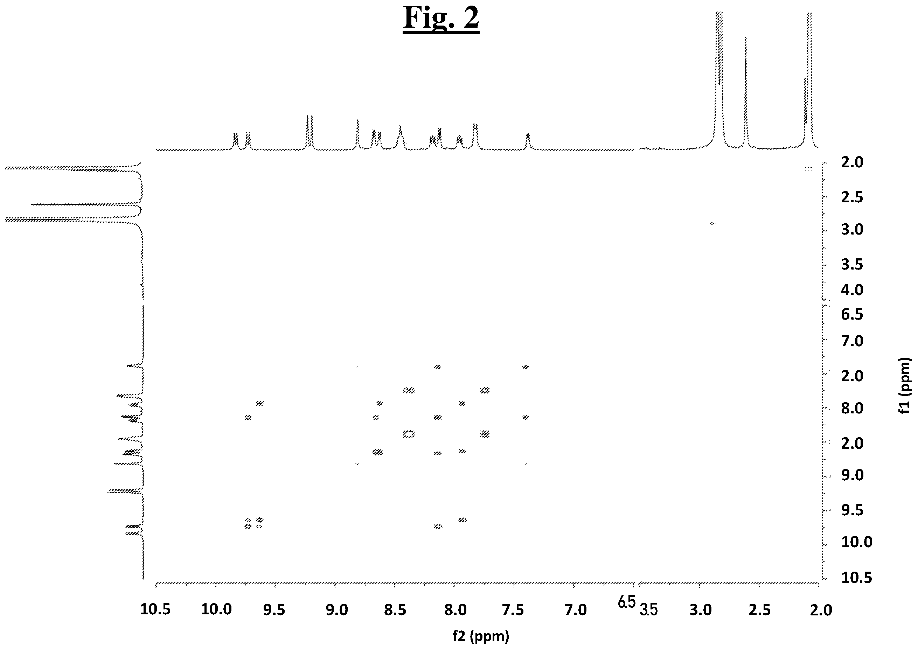

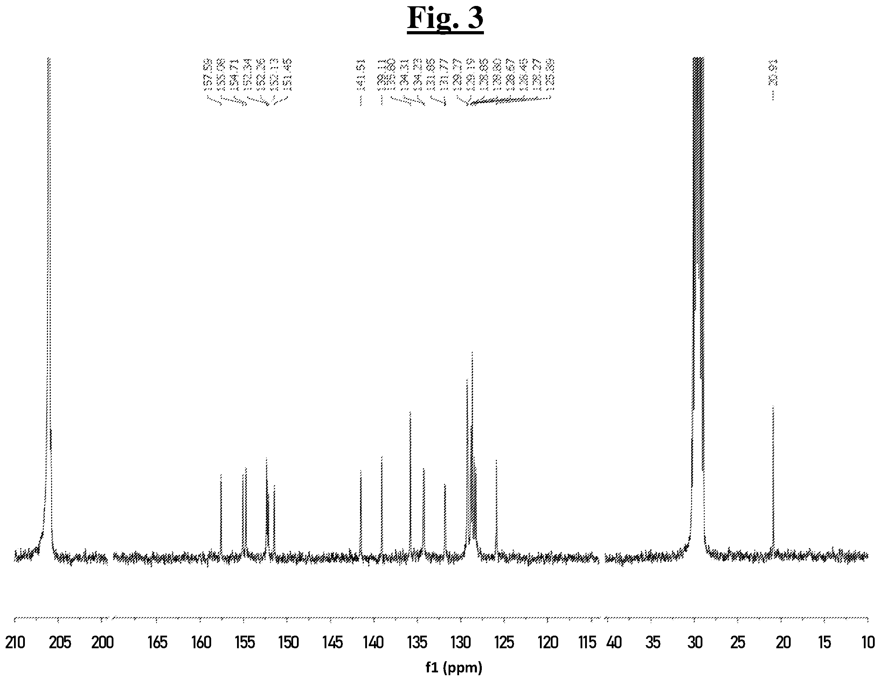

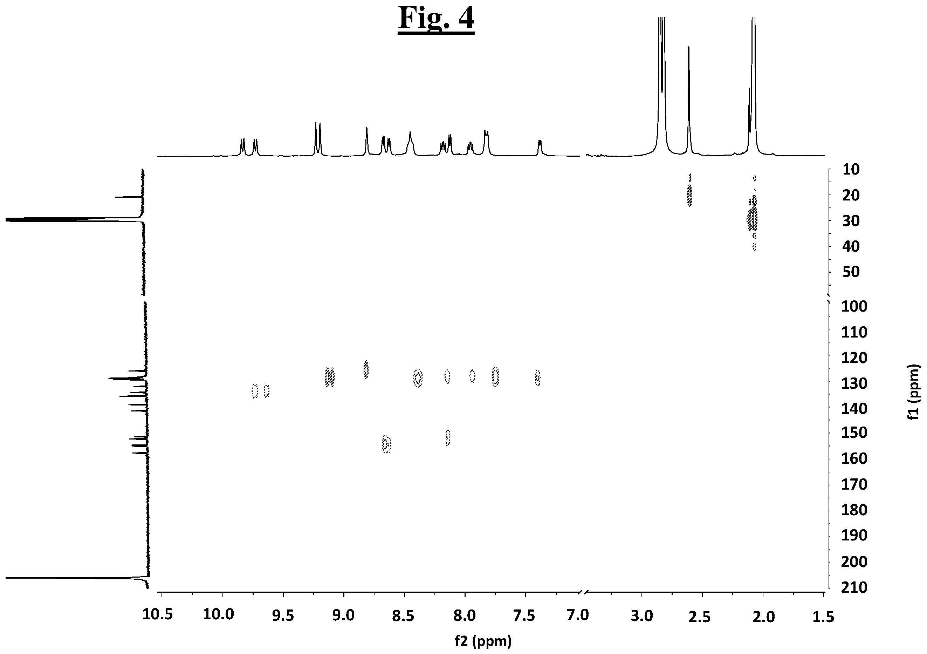

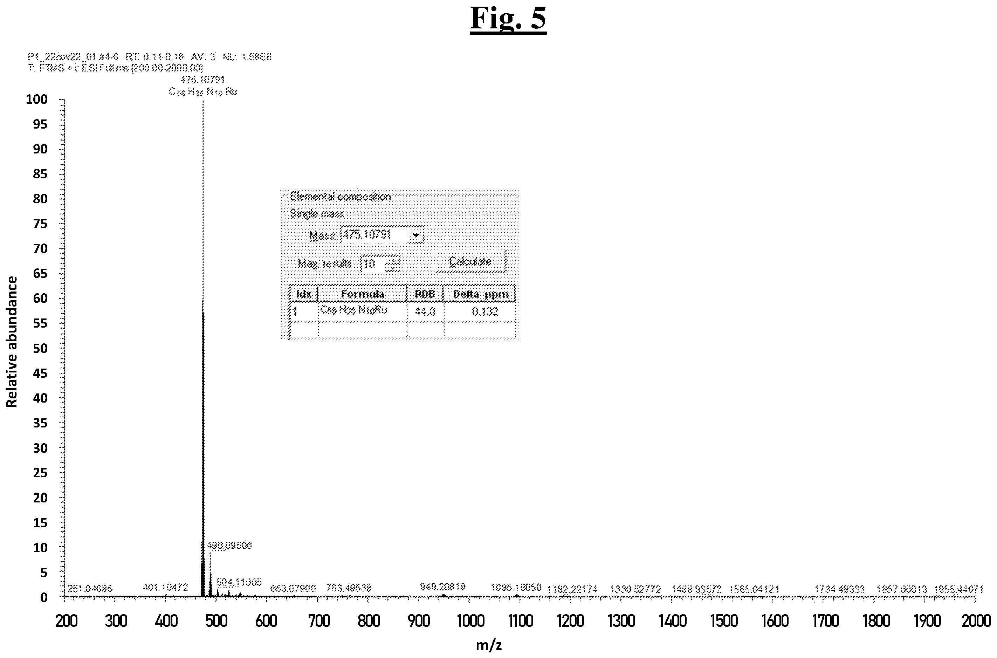

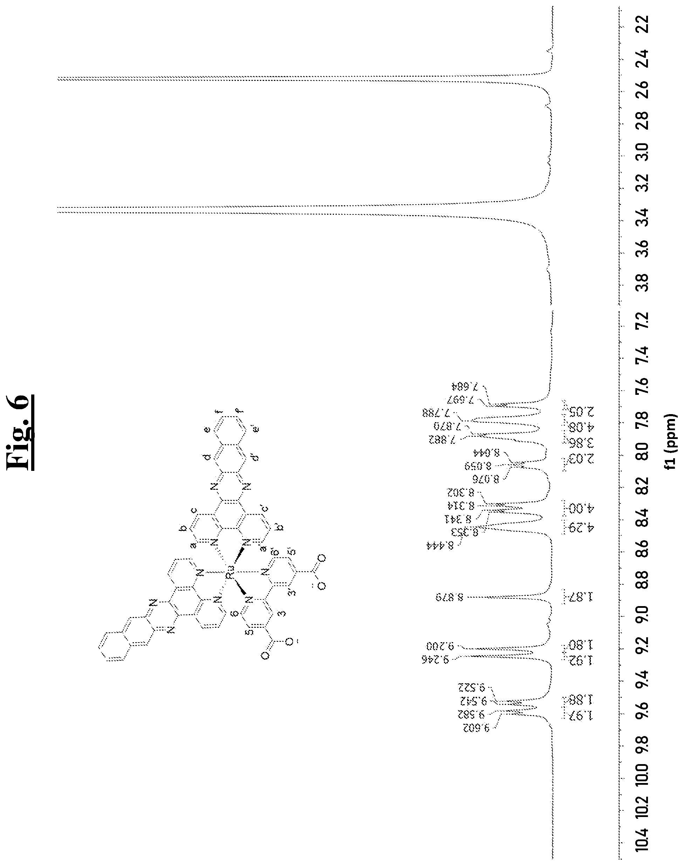

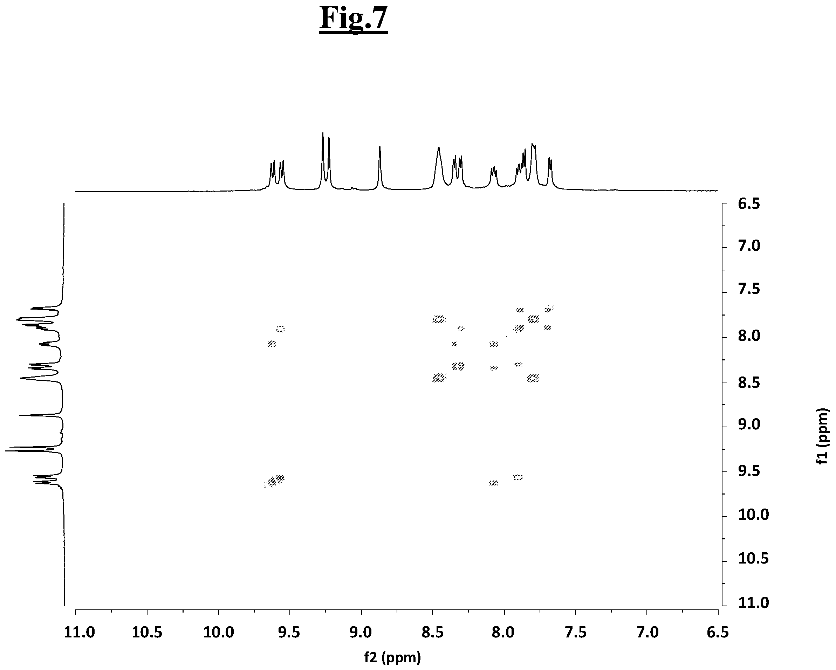

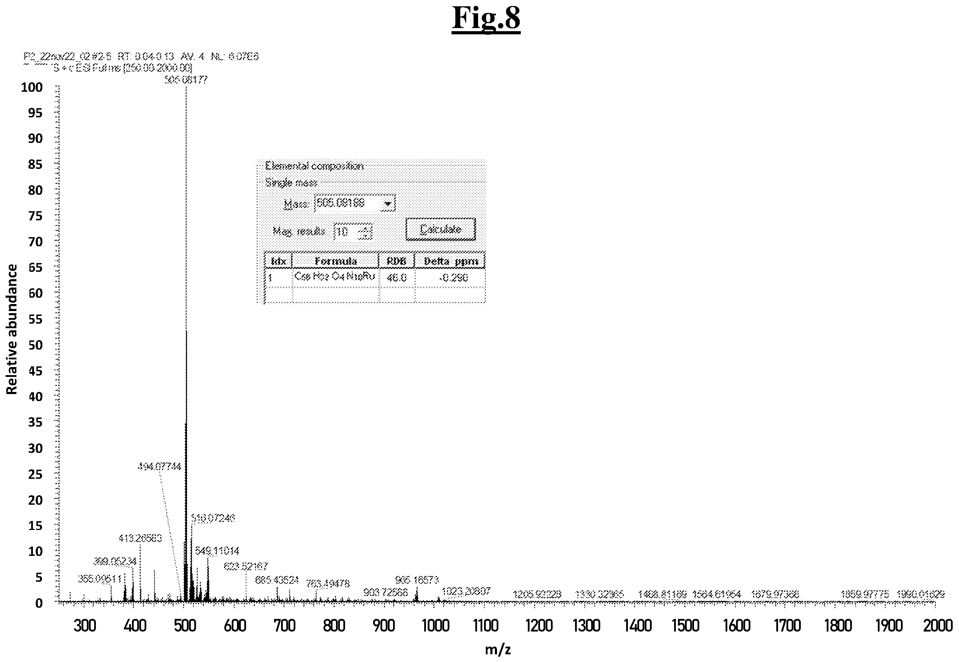

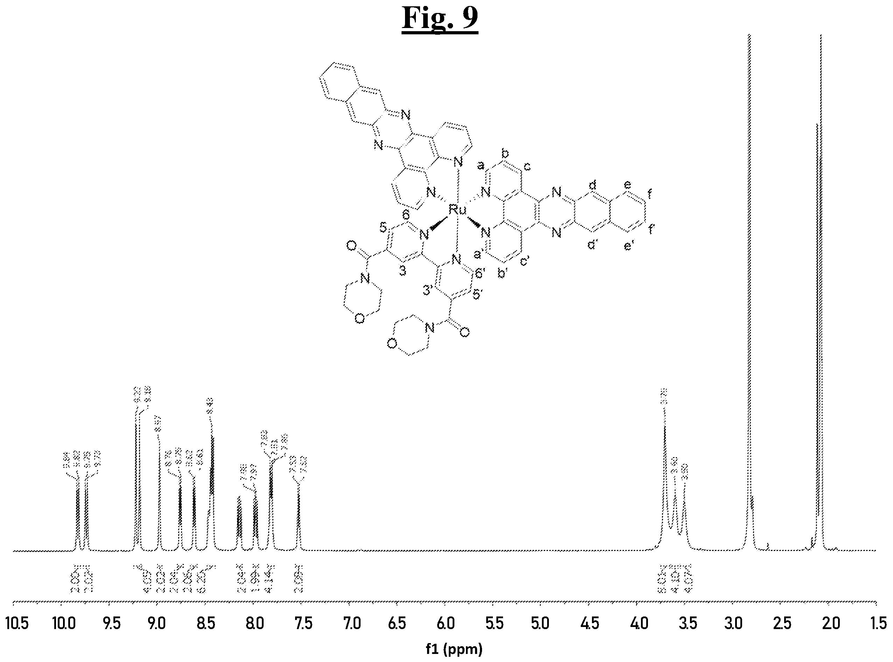

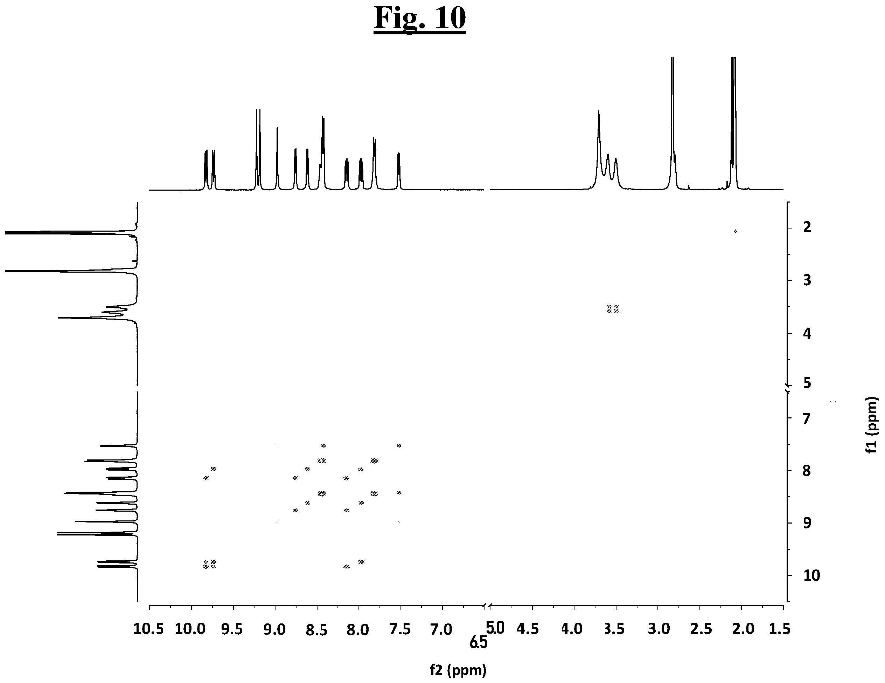

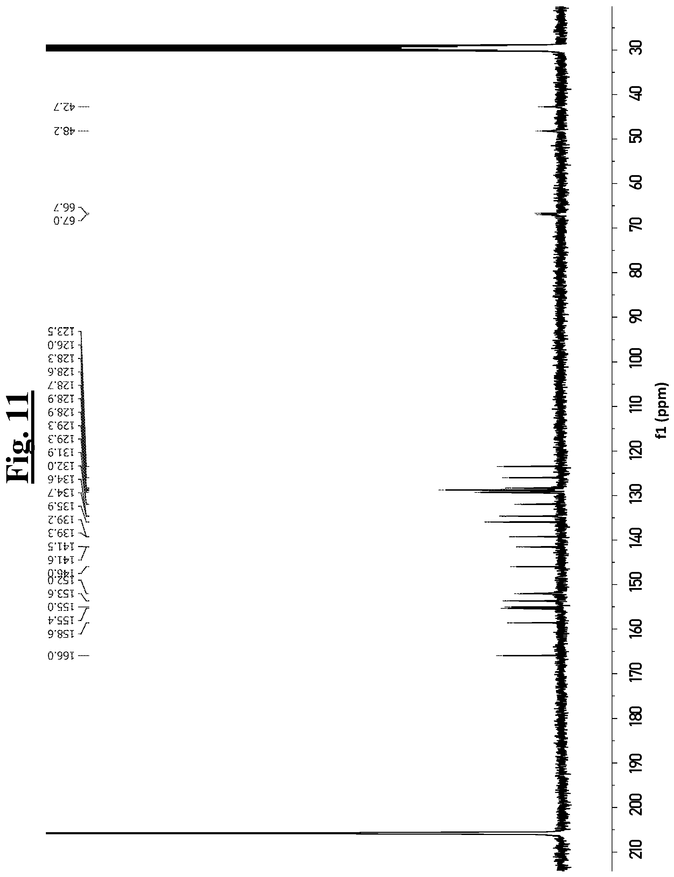

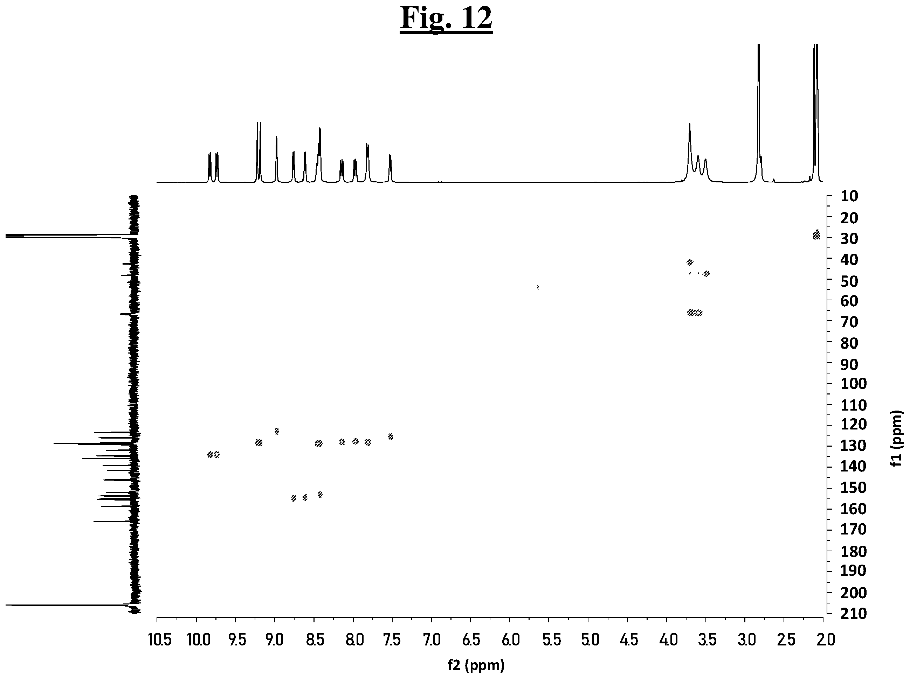

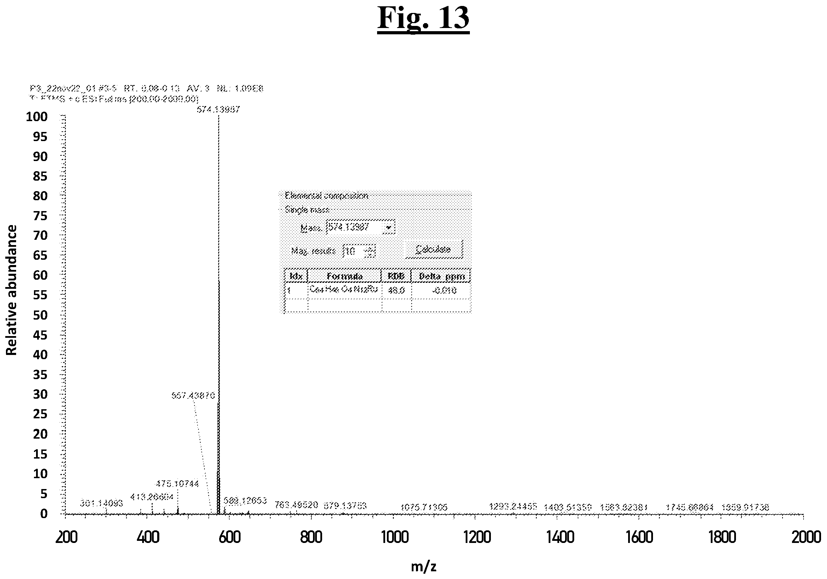

The NMR characterization data of the three complexes IIIa-IIIc synthesized as described above are shown below. The NMR characterization spectra of the three complexes are shown in Figs. 1-13. The NMR spectra were recorded using a Bruker Advance 400 MHz.

### Complex IIIa

¹H-NMR (400 MHz, (CD₃)₂CO): δ 9.83 (d, J_{c-b}=8.0 Hz, 2H, **Hc**), 9.73 (d, J_{c'-b'}=8.0 Hz, 2H, **Hc'**), 9.23 (s, 2H, **Hd**), 9.20 (s, 2H, **Hd'**), 8.81 (s, 2H, **H3/H3'**), 8.67 (d, J=4.0 Hz, 2H, **Ha**), 8.62 (d, J_{a-b}=4.0, 2H, **Ha'**), 8.49-8.44 (m, 4H, **He/He'**), 8.18 (dd, J¹_{b-c}=8.0 Hz J²_{b-a} =4.0 Hz, 2H, **Hb**), 8.12 (d, J_{H2-H3}=4.0 Hz, 2H, **H6/H6'**), 7.95 (dd, J¹_{b'-c'}=8.0 Hz J² _{b'-a'}=4.0 Hz, 2H, **Hb'**), 7.87-7.80 (m, 4H, **Hf/Hf'**), 7.38 (d, J _{H3-H2}=4.0 Hz, 2H, **H5**/**H5'**), 2.61 (s, 6H, -CH₃) ppm.

¹³C-NMR (100 MHz, (CD₃)₂CO): δ 157.59, 155.08, 154.71, 152.34, 152.26, 152.13, 151.45, 141.51, 139.11, 135.80, 134.31, 134.23, 131.85, 131.77, 129.27, 129.19, 128.85, 128.80, 128.67, 128.45, 128.27, 125.89, 20.91 ppm.

HR-MS (ESI+) m/z: calcd. for C₅₆H₃₆N₁₀Ru [M-2PF₆⁻]²⁺ 475.10791 found: 475.10791.

Anal. calcd for C₅₆H₃₆F₁₂N₁₀P₂Ru: C 54.24, N 11.30, H 2.93; found C 54.00, N 10.53, H 3.24

### Complex IIIb

¹H-NMR (400 MHz, DMSO): δ 9.62 (d, J_{c-b}=8.0 Hz Hz, 2H, **Hc**), 9.56 (d, J_{c'-b'}=8.0 Hz, 2H, **Hc'**), 9.27 (s, 2H, **Hd**), 9.22 (s, 2H, **Hd'**), 8.87 (s, 2H, **H3/H3'**), 8.50-8.40 (m, 2H, **Hf** and **Hf'**), 8.35 (d, J_{a-b}=8.0 J_{a-b}=4.8 Hz, 2H, **Ha**), 8.31 (d, J_{a'-b'}=4.8 2H, **Ha'**) 8.08 (dd, 2H, **Hb** ), 7.93-7.84 (m, 4H, **Hb'** and **H6/H6'**), 7.82-7.75 (m, 4H, **He** and **He'**), 7.68 (d, J=5.2 Hz, 2H, **H5/H5'**) ppm.

The low solubility (0.5 mM in DMSO) prevented the recording of experiments ¹³C-NMR.

HR-MS (ESI+) m/z: calcd. for C₅₆H₃₂N₁₀O₄Ru [M-2PF₆⁻]²⁺ 505.08188, found: 505.08177.

Anal. calcd for C₅₆H₃₀N₁₀O₄Ru: C 66.73, N 13.90, H 3.00; found C 62.56, N 12.62, H 3.89.

### Complex IIIc

¹H-NMR (400 MHz, (CD₃)₂CO): δ 9.83 (d, J_{c-b} = 8Hz, 2H, **Hc**), 9.74 (d, J_{c'-b'}= 8Hz, 2H, **Hc'**), 9,22 (s, 2H, **Hd**), 9.18 (s, 2H, **Hd'**), 8.98 (s, 2H, **H3/H3'**), 8.76 (d, J_{a-b}=4 Hz, 2H, **Ha**), 8.62 (d, J_{a'-b'}= 4 Hz, 2H, **Ha'**), 8.48-8.40 (m, 6H, **H6/H6', He/He'**), 8.15 (dd, J¹_{b-c} = 4 Hz, J²_{b-a} = 8 Hz, 2H, **Hb**), 7,97 (dd, J¹_{b'-c'} = 4 29 Hz, J²_{b'-a'} = 8 Hz, 2H, **Hb'**), 7.85-7.79 (m, 4H, **Hf/Hf'**), 7.53 (d, J _{H3-H2}= 4Hz, 2H, **H5/H5'**), 3.71 (bs, 8H, -CH2 morpholine), 3.60 (bs, 4H, -CH2 morpholine), 3.50 (bs, 4H, -CH2 morpholine) ppm.
¹³C-NMR (100 MHz, (CD₃)₂CO): δ165,4; 157,9; 154,8; 154,4; 153,1; 151,4; 145,4; 140,9; 140,9; 138,6; 135,3; 134,1; 134,1; 131,4; 131,3; 128,7; 128,3; 128,2; 128,1; 128,1; 127,7; 125,4; 122,9 ppm.

HR MS (ESI+) m/z: calcd. for C₆₄H₄₆N₁₂O₄Ru [M-2PF₆⁻]²⁺ 574.13987, found: 574.13897.

Anal. calcd for C₆₄H₄₆N₁₂F₁₂O₄P₂Ru: C 53.45, N 11.69, H 3.22; found C 53.28, N 11.04, H 3.42.

### Characterisation of synthesised complexes: UV-Vis absorption profiles

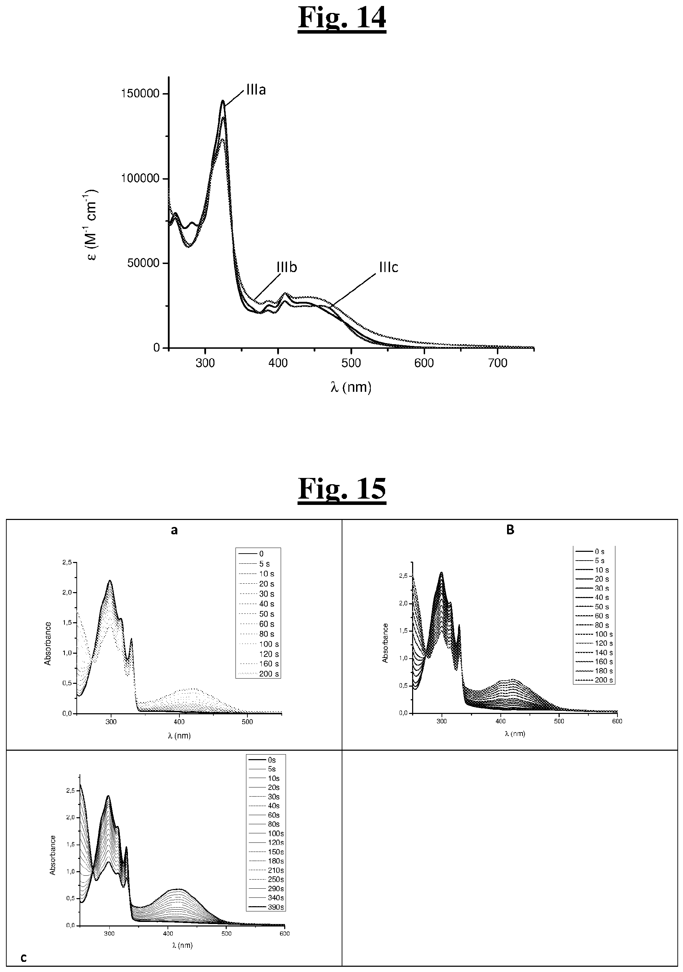

As shown in Table 1, the complexes of formula III show a broad MLCT transition centred between 440 and 450 nm, and diagnostic ¹π-π* transitions of the aromatic part of the ligands centred between 408 and 320 nm in acetonitrile. Fig. 14 shows the absorption spectra of complexes Illa-c in graphic form. All recorded absorption spectra shown in the present invention were recorded using a PerkinElmer Lambda 6 spectrophotometer.

### Determination of singlet oxygen quantum yield

The capacity of complexes Illa-c to efficiently generate singlet oxygen (¹O₂) upon irradiation with light is a key factor in the evaluation of their potential as photosensitising agents for applications in photodynamic therapy. In this connection, the singlet oxygen quantum yields of complexes Illa-c were determined by means of direct phosphorescence signal of ¹O₂ at 1270 nm generated by irradiation of acetonitrile solutions of the complexes and using dichlorotris(1,10-phenanthroline)ruthenium (II) chloride ([Ru(phen)₃Cl₂]) as a standard reference. A Horiba FluoroMax Plus spectrofluorometer was used for these measurements. The values are shown in Table 1.

**- Table 1 -**

| *Chemical-physical characterization of ruthenium complexes IIIa-c: absorption maxima, molar absorption coefficients and singlet oxygen quantum yields obtained through (a) direct phosphorescence signal measurements of ¹O₂ at 1270 nm and (b) indirect UV-visible measurements with DHN probe.* | | | |
|---|---|---|---|
| Solvent | Acetonitrile | | |
| Complex | λ_{abs} /nm (ε × 10 M³⁻¹ cm )⁻¹ | Φ¹O₂ | |
| | | (a) | (b) |
| IIIa | 440 (26,6) 409 (32,2) 387 (25,3) 324 (146,1) | 0.54 ± 0.06 | 0,44 |
| IIIb | 445 (30,0) 410 (32,4) 387 (27,8) 323 (123,2) | 0.50 ± 0.07 | 0,47 |
| IIIc | 455 (24,9) 408 (27,5) 384 (22,1) 325 (136,1) | 0.54 ± 0.6 | 0,42 |

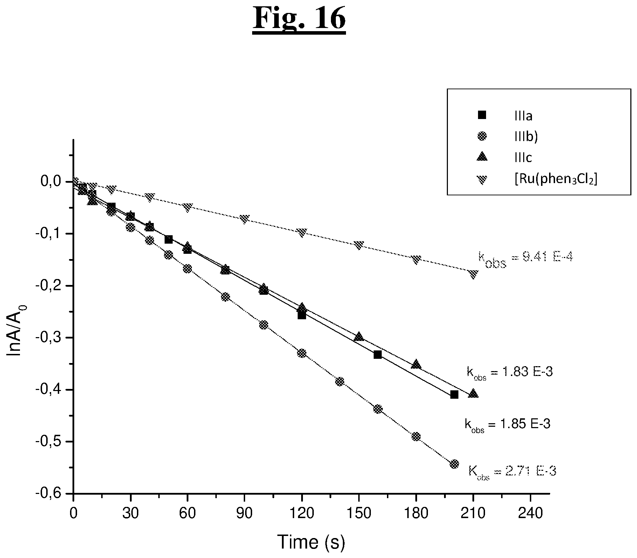

A confirmation of the production of singlet oxygen (¹O₂) with such a high quantum yields by the three IIIa-c molecular systems was obtained using 1,5-dihydroxynaphthalene (DHN) as an indirect chemical probe for ¹O₂ and [Ru(phen)₃]Cl₂ as a commercial standard. In the DHN assay, the probe is selectively and quantitatively oxidised in the presence of ¹O₂ to give the corresponding photo-oxidation product 5-hydroxy-1,4-naphthalenedione (Juglone). The production of ¹O₂ was assessed by monitoring the decrease in the DHN absorption band at λ= 297 nm and the corresponding increase in the Juglone band at λ= 427 nm. Fig. 15 shows the UV-Vis titrations obtained for IIIa-c in acetonitrile subjected to increasing irradiation times using an LED lamp with an emission spectrum between 400 and 500 nm. As shown in Fig. 15, irradiation of Illa-c results in a strong decrease in the absorption of DHN, along with a simultaneous increase in the Juglone absorption band. The rate constants for the photo-oxidation process (k_{obs}) are obtained by plotting the semilogarithmic graph of In(Aₜ/Aₒ) against the studied irradiation times, as shown in Fig. 16, in comparison with that found for [Ru(phen)₃]Cl₂ under the same experimental conditions. On this basis, the quantum yield of formation of ¹O₂ can be obtained via the equation ${φ}_{Δ} = \frac{k {I}_{abs ST .}}{{k}_{ST .} {I}_{abs}} {φ}_{Δ ST} ,$ where ϕ_{Δ} are the quantum yields of ¹O₂ production from the photosensitising agents, k are the rate constants obtained from the DHN assays, I_{abs} represents the absolute value for the integration of the absorption of the photosensitising agents in the spectral emission region of the LED lamp (400 nm to 500 nm), and the subscript ST indicates the commercial standard [Ru(phen)₃]Cl₂.

Considering that the standard quantum yield of ¹O₂ production by [Ru(phen)₃]Cl₂ is 0.38 and that the k_{obs} obtained are 1.85 × 10⁻³ for complex IIIa, 2.71 × 10⁻³ for complex IIIb, 1.83 × 10⁻³ for complex IIIc and 9.41 × 10⁻⁴ for the reference (Fig. 16), the quantum yields of ¹O₂ production for complexes Illa-c are 0.44, 0.47 and 0.42, respectively, which are agreement with those obtained by direct measurement of the phosphorescence signal (Table 1).

### Interaction with calf thymus DNA

The ability of complexes IIIa-c to bind DNA was assessed on *calf thymus* DNA (ct-DNA) monitoring the changes in the UV-Vis spectra of the 322 nm band upon titration of a solution of the complex at a fixed concentration (10 µM) in a buffer solution (Tris-HCl 10 mM, NaCl 50 mM, pH 7.2) with increasing amounts of biopolymer. At each addition, samples were incubated at room temperature during 5 minutes and UV-Vis spectra were recorded using a Tris-HCl buffered solution containing equal amounts of ct-DNA as a blank in order to eliminate the intrinsic contribution to absorbance of the ct-DNA itself. The binding constants found for complexes Illa-c are shown in Table 2.

**- Table 2 -**

| *Binding constants (K_{b}) of ruthenium complexes synthesized in this invention with ct-DNA* | |
|---|---|
| | Kb (ct- DNA) |
| IIIa | 7,49 × 105 |
| IIIb | 2,34 × 106 |
| IIIc | 8,75 × 105 |

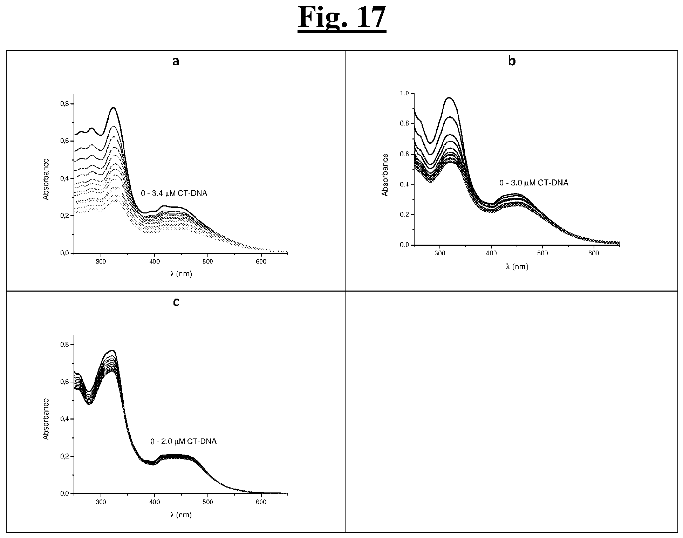

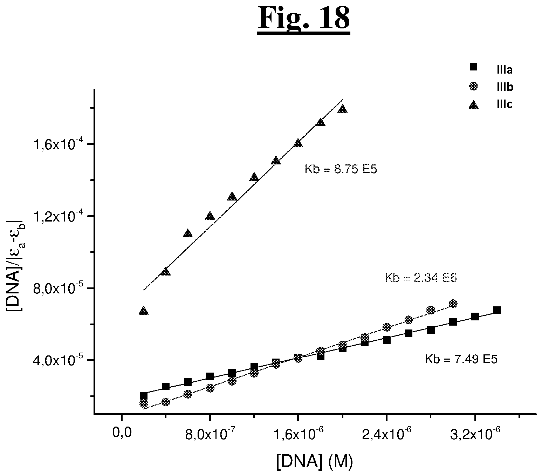

As shown in Fig. 17, the consecutive addition of ct-DNA (0 to 3.4 µM) results in a hypochromism of the whole absorption spectrum of complexes IIIa-c. The intrinsic binding constant K_{b} of complexes IIIa-c with ct-DNA was determined using the equation $\frac{\left[DNA\right]}{\left[{ε}_{a}-{ε}_{f}\right]} = \frac{\left[DNA\right]}{\left({ε}_{b}-{ε}_{f}\right)} + \frac{1}{{K}_{b} \left({ε}_{b}-{ε}_{f}\right)}$ where [DNA] is the concentration of ct-DNA in base pairs, and the apparent adsorption coefficients εₐ, ε_{f} and ε_{b} are A_{obs}/[Ru], the molar extinction coefficient for the unbound complex and the molar extinction coefficient for the Ru complexes in their fully DNA-bound form, respectively. The binding constants (K_{b}) are obtained as the ratio of the slope and the intercept of the linear regression of [DNA]/|εₐ -ε_{f} / plotted against [DNA], as shown in Fig. 18.

### Activity of compound IIIa

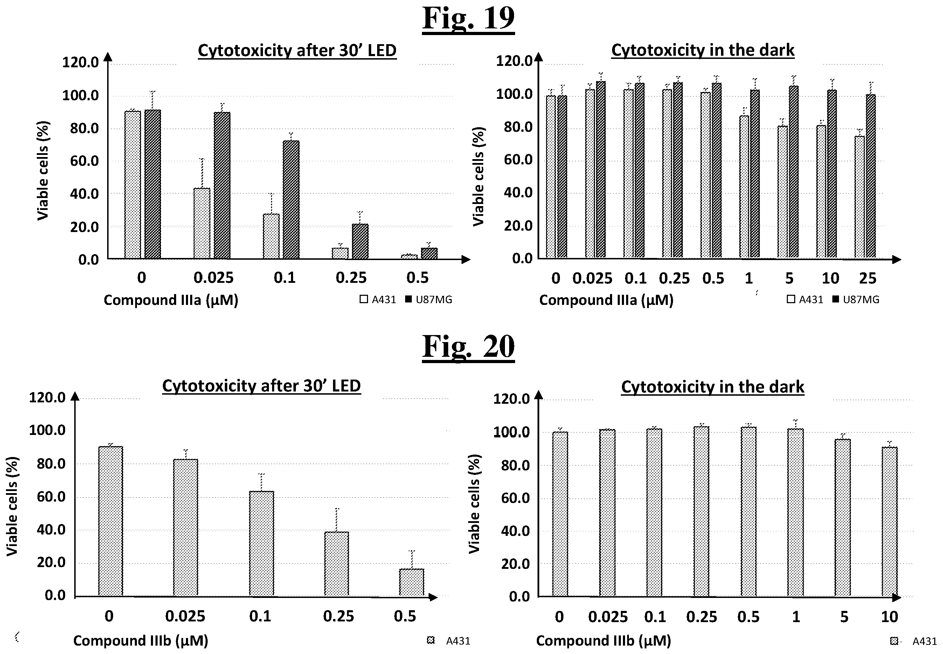

The activity of compound IIIa as a photosensitising agent in photodynamic therapy was evaluated on two in-vitro tumour models: squamous cell carcinoma of the epidermis (A431) and glioblastoma multiforme (U87MG). Cells cultured on 96-well plates were treated with increasing concentrations of Illa dispersed in the culture medium for one hour. At the end of the incubation, the medium was replaced, and the cells were exposed to LED light (λₘₐₓ 462 nm) for 30 minutes, or were left in the dark during the same time. At the end of the 30 minutes, the cells were placed back and maintained in the incubator for 24 hours before measuring the metabolic activity by MTT assay. Six replicates were used for each condition, and cell viability of 100% was calculated from the response of untreated cells not exposed to LED light. On both models, compound Illa showed high potency and specificity. In particular, compound IIIa induced extensive cell death after LED irradiation even at nanomolar concentrations, while exhibiting little or no toxicity when it was not activated by light, as shown in Fig. 19. The high specificity of Illa, defined as toxicity only when exposed to light, is evident from the difference in IC₅₀ values, which indicate a potent cytotoxic activity in the presence of light irradiation and substantial inertia in the dark at the concentrations tested, as shown in Table 3. This indicates that compound Illa has a great potential as a photosensitising agent in the photodynamic therapy for treating various types of tumours.

**- Table 3 -**

| *Mean IC₅₀ values obtained from in-vitro cytotoxicity experiments on compound IIIa tumour models* | | |
|---|---|---|
| Cell cultures | IC₅₀ (maximum response set to zero) | |
| | 30' LED light exposure | In the dark |
| A431 | 0.0253 µM | > 40 µM |
| U87MG | 0.164 µM | > 40 µM |

### Activity of compound IIIb

The activity of compound IIIb as a photosensitising agent in photodynamic therapy was evaluated on an in vitro tumour model, i.e., squamous cell carcinoma of the epidermis (A431), following the same protocol as compound IIIa. Compound IIIb showed high potency and specificity. In particular, IIIb induced extensive cell death after LED irradiation even at nanomolar concentrations, while exhibiting little or no toxicity when it was not activated by light, as shown in Fig. 20. The high specificity of IIIb, defined as toxicity only when exposed to light, is evident from the difference in IC₅₀ values, which indicate a potent cytotoxic activity in the presence of light irradiation and substantial inertia in the dark at test concentrations, see Table 4. This indicates that compound IIIb has a great potential as a photosensitising agent in the photodynamic therapy for treating various types of tumours.

**- Table 4 -**

| *Mean IC₅₀ values obtained from in vitro cytotoxicity experiments on compound IIIb tumour models* | | |
|---|---|---|
| Cell cultures | IC₅₀ (maximum response set to zero) | |
| | 30' LED light exposure | In the dark |
| A431 | 0.191 µM | > 40 µM |

### Formulation of complex IIIb-loaded cubosomes: chemical-physical characterisation

The formulation of cubosomes loaded with complex IIIb was prepared using monoolein, i.e. glycerol monooleate (1-monooleoylglycerol, RYLO MG 19 PHARMA, 98.1 wt.%, supplied by Danisco A/S), and Pluronic F108 (PEO132-PPO50-PEO132, purchased from Sigma Aldrich) as stabilising agent. Fresh distilled water purified with a Milli-Q system (Millipore) was used to prepare each sample and it was filtered through a 0.22 µm pore size hydrophilic filter before each sample preparation was used.

Cubosomes were prepared by melting the monoolein at 40°C and dispersing the melted monoolein with the help of an ultrasonic bath. An appropriate amount of an aqueous solution of Pluronic F108 stabiliser was then added to the lipid phase, and the mixture was subjected to ultrasonication with a UP100H ultrasonic processor developed by Hiescher (90% amplitude; 1 s ON, 1 s OFF) for cycles of 5, 4, 3, 2 and 1 min.

The average hydrodynamic diameter and polydispersity index, as a measure of the width of the particle size distribution, were determined by Dynamic Light Scattering using a nano Zetasizer (Malvern Instrument). The samples were backscattered using a helium-neon laser (633 nm) at an angle of 173° and at a constant temperature of 25°C. The zeta-potential was estimated with the nano Zetasizer using the M3-PALS (Phase Analysis Light Scattering) technique.

The macroscopic appearance of the obtained sample was that of a fluid, opaque, pale orange aqueous dispersion, due to the presence of the Ruthenium complex. The investigation of the complex IIIb-loaded cubosomes started by assessing the encapsulation efficiency of the ruthenium complex. To this purpose, the complex IIIb-loaded cubosomes were separated from the free complex IIIb by dialyzing the formulation through a 14 kDa molecular weight cutoff tubular cellulose membrane (by Sigma Aldrich) against 2 L of water for 2 h, changing the water after one hour, at room temperature. Then, after disintegration of the formulation in methanol, in which all components are soluble, using a Synergy 4 multiplate reader (BioTek, Winooski, USA), the encapsulation/entrapment efficiency was evaluated by UV-Visible spectroscopy at 325 nm. Specifically, this efficiency was calculated by the expression: $Encapsulation efficiency = \frac{mass of drug after dialysis}{mass of weighted drug} \times 100 %$

The final concentration of Illb was 0.02% (w/w) with an encapsulation efficiency value of 60 ± 5 %. Therefore, the final composition of complex IIIb-loaded cubosomes was
monoolein/ Pluronic F108/ complex IIIb/ water = 3.30 / 0.03 / 0.02 / 96.65 % (w/w).

The samples were visually inspected before any measurement by Dynamic Light Scattering measurement to check the absence of aggregates or phase separation. Moreover, the colloidal system was characterised by nanoparticles with an average diameter of 142 ± 1 nm, a polydispersity index of 0.13 ± 0.01 and a zeta potential of -30 ± 2 mV.

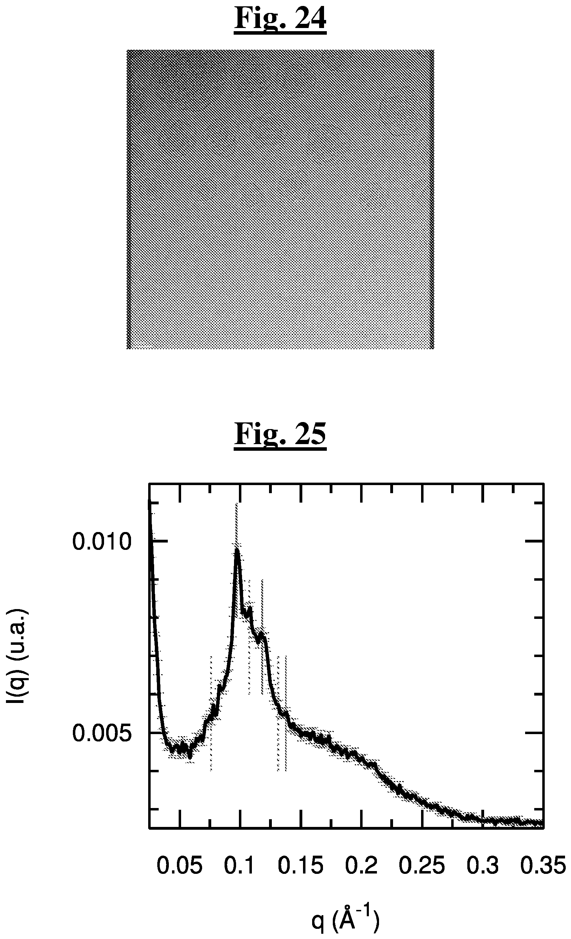

The morphology of IIIb-loaded cubosomes was revealed by transmission electron microscopy at cryogenic temperatures (Cryo-TEM). As shown in Fig. 24, the cubosomes appear as spherical nanoparticles of different sizes with an inner structure characterised by a dark matrix and alternating bright spots, representing the lipid bilayer and water channels, respectively.

The inner nanostructure of complex IIIb-loaded cubosomes was assessed by means of Small Angle X-ray Scattering (SAXS) experiments. In particular, the recorded SAXS diffractogram of complex IlIb-loaded cubosomes, shown in Fig. 25, strongly suggests the simultaneous presence of two bicontinuous cubic phases, Pn3m and Im3m, characterised by lattice parameters of 92 ± 1 Å and 117 ±1 Å and water channel radii of 38 ± 1 Å and 37 ± 1 Å, respectively.

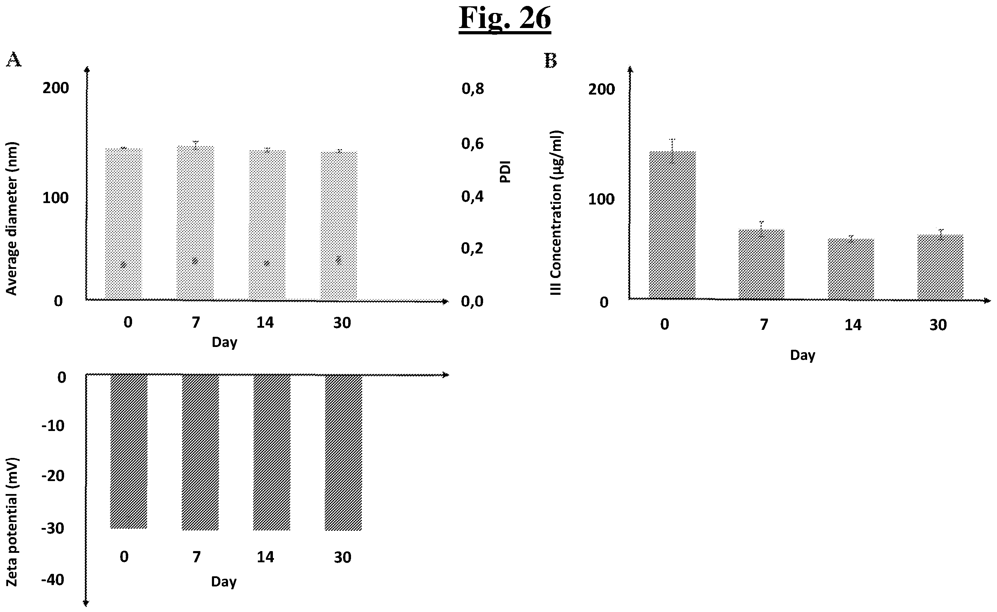

Information on the formulation stability was obtained by monitoring the mean diameter, the polydispersity index PDI and the zeta potential over a period of 30-day, as shown in Fig. 26A. Moreover, the chemical stability of complex IIIb encapsulated in the cubosomes was evaluated by analysing the formulation through UV-Visible spectroscopy, as shown in Fig. 26B. The study of the particle size distribution revealed an optimal stability of the formulation, as shown in Fig. 26.

Indeed, the mean diameter did not appreciably change during the 30-day storage at 25°C, showing a value around 140 nm throughout the study. The polydispersity index remained almost constant and below 0.15, which confirms t a retention of the fairly narrow size distribution during storage. Moreover, the value of the zeta potential did not change, with recorded values of ca. -30 mV. In addition, UV-Visible analysis revealed a decrease in the complex IIIb concentration between day 0 and day 7. However, the complex IIIb concentration remained unchanged from day 7 until the end of the investigation.

### Activity of compound IIIb encapsulated in cubosomes

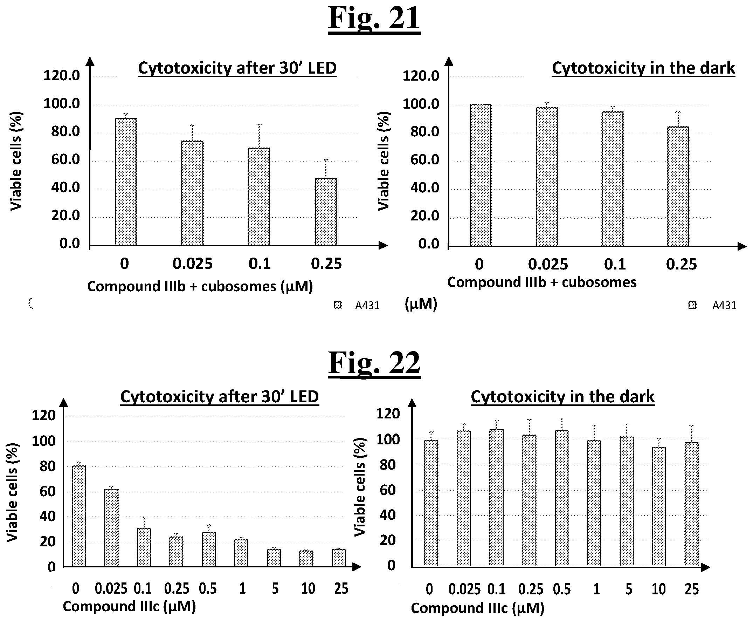

The activity of compound IIIb encapsulated in cubosomes as a photosensitising agent in photodynamic therapy was evaluated on an *in vitro* tumour model: squamous cell carcinoma of the epidermis (A431), following the same protocol as compound Illa and IIIb as such. Compound IIIb encapsulated in cubosomes induced extensive cell death after LED irradiation even at nanomolar concentrations, while exhibiting little or no toxicity when it was not activated by light, as shown in Fig. 21. The encapsulation in cubosomes leads to a reduced IC₅₀ value, in comparison with IIIb as a free compound (Tables 4 and 5). However, the incorporation into a delivery system can bring numerous benefits, including increased distribution in the tumour after in vivo administration, increased stability and solubility, and improved crossing of biological barriers.

**- Table 5 -**

| *Mean IC₅₀ values obtained from in vitro cytotoxicity experiments on tumour models of compound IIIb encapsulated in cubosomes* | | |
|---|---|---|
| Cell cultures | IC₅₀ (maximum response set to zero) | |
| | 30' LED light exposure | In the dark |
| A431 | 0.367 µM | ND |

Moreover, it should be noted that the medicament described above represents the first example of a Ru(II) polypyridyl complex encapsulated in cubosomes reported in the literature to date.

### Activity of compound IIIc

The activity of compound IIIc as a photosensitising agent in photodynamic therapy was evaluated on an in-vitro tumour model, Methastatic Lung Adenocarcinoma (Calu-3), following the same protocol as compounds Illa and IIIb. Compound IIIc showed high potency and specificity. In particular, IIIc induced extensive cell death after LED irradiation even at nanomolar concentrations, while exhibiting little or no toxicity when it was not activated by light, as shown in Fig. 22. The high specificity of IIIc, defined as toxicity only when exposed to light, is evident from the difference in IC50 values, which indicate a potent cytotoxic activity in the presence of light irradiation and substantial inertia in the dark at test concentrations, see Table 6. This indicates that compound IIIc has a great potential as a photosensitising agent in the photodynamic therapy for treating various types of tumours.

**- Table 6 -**

| *Mean IC50 values obtained from in-vitro cytotoxicity experiments on compound IIIc tumour models* | | |
|---|---|---|
| Cell cultures | IC50 (maximum response set to zero) | |
| | 30' LED light exposure | In the dark |
| Calu-3 | 0.0639 µM | > 40 µM |

### Activity of compound IIIc encapsulated in cubosomes

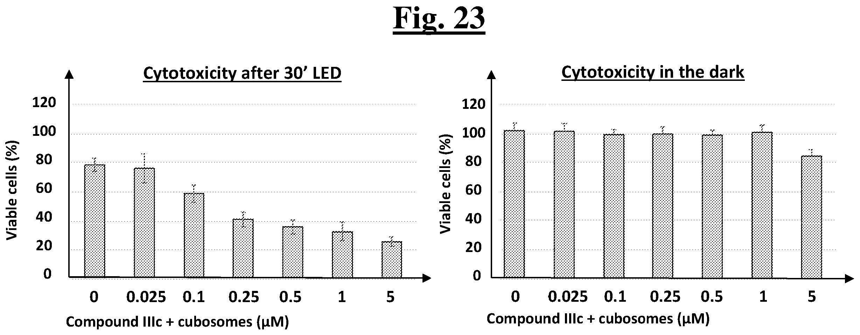

The activity of compound IIIc encapsulated in cubosomes as a photosensitising agent in photodynamic therapy was evaluated on an in vitro tumour model: Methastatic Lung Adenocarcinoma (Calu-3), following the same protocol as compound Illa and IIIb. Compound IIIc encapsulated in cubosomes induced extensive cell death after LED irradiation even at nanomolar concentrations, while exhibiting little or no toxicity when it was not activated by light, as shown in Fig. 23. The encapsulation in cubosomes leads to a reduced IC50 value, in comparison with IIIc as a free compound (Tables 6 and 7). However, the incorporation into a delivery system can bring numerous benefits, including increased distribution in the tumour after in vivo administration, increased stability and solubility, and improved crossing of biological barriers.

**- Table 7 -**

| *Mean IC50 values obtained from in vitro cytotoxicity experiments on tumour models of compound IIIc encapsulated in cubosomes* | | |
|---|---|---|
| Cell cultures | IC50 (maximum response of zero) | |
| | 30' LED light exposure | In the dark |
| Calu-3 | 0.144 µM | > 5 µM |

### BIBLIOGRAPHICAL REFERENCES

1) Wang, L. et al. "π-Expansive Heteroleptic Ruthenium (II) Complexes as Reverse Saturable Absorbers and Photosensitizers for Photodynamic Therapy" Inorg. Chem. 56, 3245-3259 (2017).
2) Turro et al., "Excited state dynamics of two new Ru(II) cyclometallated dyes: Relation to cells for solar energy conversion and comparison to conventional systems" J. Phys. Chem. C (2012).

## Claims

1. Ruthenium (II) coordination compounds having formula: 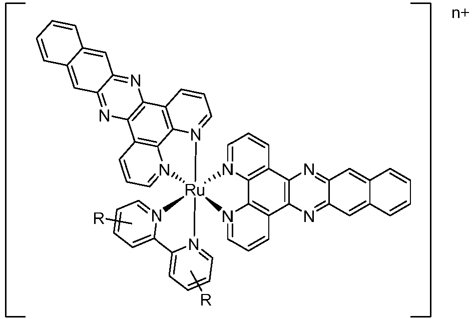
wherein n is selected between 0 and 2,
wherein R is a substituent group selected from the group consisting of:
- a methyl group;
- a phenyl group;
- a -CH₂NH₂ group;
- a -CH₂OH group;
- a carboxyl group -COOH;
- an ester group -COOR₁,
where R₁ is selected from the group consisting of:
- a linear or branched alkyl group with a number of carbon atoms in the range from 1 to 4;
- an unsubstituted benzyl group;
- a polyethylene glycol methoxy group H(OCH₂CH₂)₁₋₄OH;
- an amide group -CONR₂R₃,
where
- R₂ is hydrogen and R₃ is a group selected from the group consisting of:
- a linear or branched alkyl group with a number of carbon atoms comprised in the range from 1 to 4;
- a -(CH₂)₂NH₂ group;
- a phenyl group,
or
- R₂ and R₃ are linear or branched alkyl groups with a number of carbon atoms in the range from 1 to 4,
or
- NR₂R₃ is a group selected from the group consisting of:
- a cycloalkyl secondary amine with a number of carbon atoms in the range from 4 to 5;
- a heterocycloalkyl secondary amine comprising a heteroatom selected between nitrogen and oxygen,
for use as photosensitising agents in a photodynamic therapy for treating neoplasms.

2. The coordination compounds for use according to claim 1, wherein each substituent group R is bonded to a carbon atom at a position selected between the 5,5' position and the 4,4' position of each pyridine ring of the 2,2'-bipyridine group:

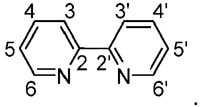

3. The coordination compounds for use according to claim 1, wherein said substituent groups R are bonded to carbon atoms in symmetrical 5,5' or 4,4' positions of respective pyridine rings of the 2,2'-bipyridine group

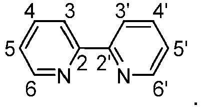

4. A photosensitizer medicament for photodynamic therapy to treat neoplasms comprising a ruthenium (II) coordination compound as a photosensitising agent, wherein said ruthenium (II) coordination compound has the formula: 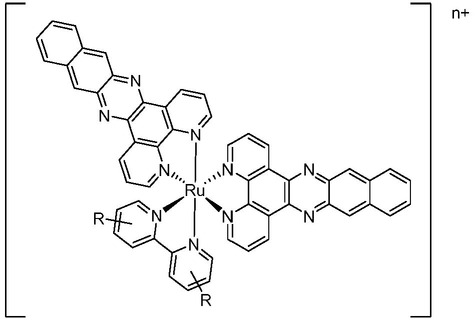
wherein n is selected between 0 and 2,
wherein R is a substituent group selected from the group consisting of:
- hydrogen;
- a methyl group;
- a phenyl group;
- a -CH₂NH₂ group;
- a -CH₂OH group;
- a carboxyl group -COOH;
- an ester group -COOR₁,
where R₁ is selected from the group consisting of:
- a linear or branched alkyl group with a number of carbon atoms in the range from 1 to 4;
- an unsubstituted benzyl group;
- a polyethylene glycol methoxy group H(OCH₂ CH₂) ₁₋₄OH;
- an amide group -CONR₂R₃,
where
- R₂ is hydrogen and R₃ is a group selected from the group consisting of:
- a linear or branched alkyl group with a number of carbon atoms comprised in the range from 1 to 4;
- a -(CH₂) ₂NH₂ group;
- a phenyl group,
or
- R₂ and R₃ are linear or branched alkyl groups with a number of carbon atoms in the range from 1 to 4,
or
- NR₂R₃ is a group selected from the group consisting of:
- a cycloalkyl secondary amine with a number of carbon atoms in the range from 4 to 5;
- a heterocycloalkyl secondary amine comprising a heteroatom selected between nitrogen and oxygen,
wherein said coordination compound is incorporated in liquid-crystalline lamellar and/or non-lamellar lipid nanoparticles.

5. The medicament for photodynamic therapy according to claim 4, wherein said nanoparticles are cubosomes.

6. The medicament for photodynamic therapy according to claim 4, wherein said nanoparticles are selected from the group consisting of:
- hexosomes;
- ethosomes;
- transferosomes;
- solid lipid nanoparticles;
- nanoemulsions;
- a combination of the above.

7. The medicament for photodynamic therapy according to claim 4, wherein said nanoparticles are liposomes.

8. A process for obtaining a ruthenium (II) coordination compounds having formula: 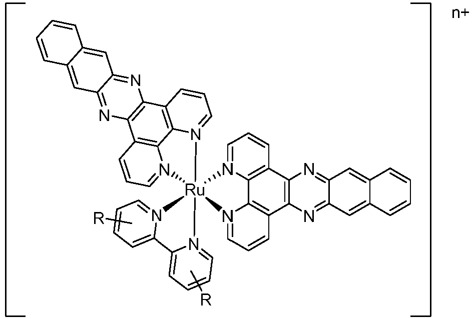
where n is selected between 0 and 2,
where R is a substituent group selected from the group consisting of:
- hydrogen;
- a methyl group;
- a phenyl group;
- a -CH₂NH₂ group;
- a -CH₂OH group;
- a carboxyl group -COOH;
- an ester group -COOR₁,
where R₁ is selected from the group consisting of:
- a linear or branched alkyl group with a number of carbon atoms in the range from 1 to 4;
- an unsubstituted benzyl group;
- a polyethylene glycol methoxy group having the formula H(OCH₂CH₂)₁₋₄OH;
- an amide group -CONR₂R₃,
where
- R₂ is hydrogen and R₃ is a group selected from the group consisting of:
- a linear or branched alkyl group with a number of carbon atoms in the range from 1 to 4;
- a -(CH₂)₂NH₂;
- a phenyl group,
or
- R₂ and R₃ are linear or branched alkyl groups with a number of carbon atoms in the range from 1 to 4,
or
- NR₂R₃ is a group selected from the group consisting of:
- a cycloalkyl secondary amine with a number of carbon atoms in the range from 4 to 5;
- a heterocycloalkyl secondary amine comprising a heteroatom selected between nitrogen and oxygen,
said process comprising the sequence of steps of:
- obtaining a reactive polymeric precursor [Ru(CO)₂Cl₂] ₘ by reaction between ruthenium (III) chloride and paraformaldehyde;
- obtaining an intermediate complex *trans*-Cl[Ru(NN)Cl₂(CO)₂]: 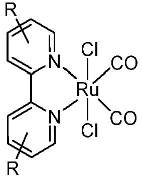
where NN is a 2,2'-bipyridine ligand symmetrically functionalised with two equal functional R groups,
by reaction of said reactive polymer precursor [Ru(CO)₂Cl₂]ₘ with a predetermined symmetrically functionalized 2,2'-bipyridine group with said two equal R functional groups,
- obtaining said ruthenium (II) coordination compounds by reaction of said intermediate complexes 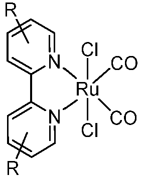 with benzo[i]dipyridine[3,2-a:2',3'-c]phenazine.

9. The process according to claim 8, wherein each substituent group R is bonded to a carbon atom at a position selected between the 5,5' position and the 4,4' position of each pyridine ring of the 2,2'-bipyridine group

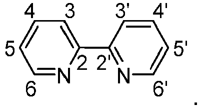

10. The process according to claim 8, wherein said substituent groups R are bonded to carbon atoms in symmetrical 5,5' or 4,4' positions of the respective pyridine rings of the 2,2'-bipyridine group

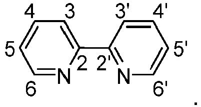

## Patentansprüche

1. Ruthenium(II)-Koordinationsverbindungen mit der Formel: 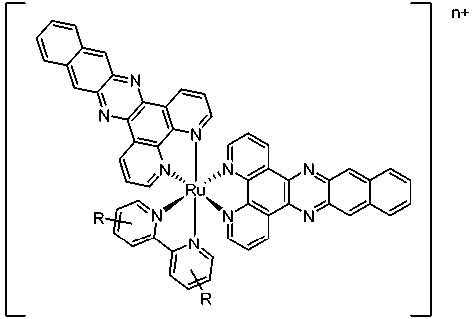
wobei n zwischen 0 und 2 ausgewählt ist,
wobei R eine Substituentengruppe ist, die aus der Gruppe ausgewählt ist, die aus Folgendem besteht:
- einer Methylgruppe;
- einer Phenylgruppe;
- einer -CH₂NH₂-Gruppe;
- einer -CH₂OH-Gruppe;
- einer Carboxylgruppe -COOH;
- einer Estergruppe -COOR₁,
wobei R₁ aus der aus Folgendem bestehenden Gruppe ausgewählt ist:
- einer linearen oder verzweigten Alkylgruppe mit einer Anzahl von Kohlenstoffatomen im Bereich von 1 bis 4;
- einer nichtsubstituierten Benzylgruppe;
- einer Polyethylenglykol-Methoxygruppe H(OCH₂CH₂)₁₋₄OH;
- einer Amidgruppe -CONR₂R₃,
wobei
- R₂ Wasserstoff ist und R₃ eine aus der aus Folgendem bestehenden Gruppe ausgewählte Gruppe ist:
- einer linearen oder verzweigten Alkylgruppe mit einer Anzahl von Kohlenstoffatomen, die im Bereich von 1 bis 4 umfasst ist;
- einer -(CH₂)₂NH₂-Gruppe;
- einer Phenylgruppe,
oder
- R₂ und R₃ lineare oder verzweigte Alkylgruppen mit einer Anzahl von Kohlenstoffatomen im Bereich von 1 bis 4 sind,
oder
- NR₂R₃ eine aus der aus Folgendem bestehenden Gruppe ausgewählte Gruppe ist:
- einem sekundären Cycloalkylamin mit einer Anzahl von Kohlenstoffatomen im Bereich von 4 bis 5;
- einem sekundären Heterocycloalkylamin, das ein unter Stickstoff und Sauerstoff ausgewähltes Heteroatom umfasst,
zur Verwendung als photosensibilisierende Mittel in einer photodynamischen Therapie zur Behandlung von Neoplasmen.

2. Koordinationsverbindungen zur Verwendung nach Anspruch 1, wobei jede Substituentengruppe R an ein Kohlenstoffatom an einer Position gebunden ist, die unter der 5,5'-Position und der 4,4'-Position jedes Pyridinrings der 2,2'-Bipyridingruppe ausgewählt ist:

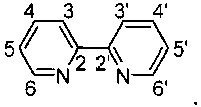

3. Koordinationsverbindungen zur Verwendung nach Anspruch 1, wobei die Substituentengruppen R an Kohlenstoffatome an symmetrischen 5,5'- oder 4,4'-Positionen jeweiliger Pyridinringe der 2,2'-Bipyridingruppe gebunden sind

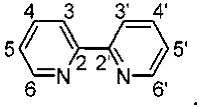

4. Photosensibilisator-Arzneimittel für eine photodynamische Therapie zur Behandlung von Neoplasmen, umfassend eine Ruthenium(II)-Koordinationsverbindung als photosensibilisierendes Mittel, wobei die Ruthenium(II)-Koordinationsverbindung die folgende Formel aufweist: 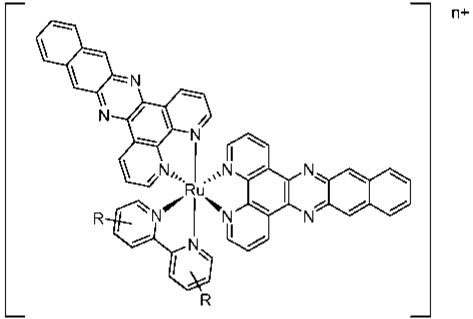
wobei n zwischen 0 und 2 ausgewählt ist,
wobei R eine Substituentengruppe ist, die aus der Gruppe ausgewählt ist, die aus Folgendem besteht:
- Wasserstoff;
- einer Methylgruppe;
- einer Phenylgruppe;
- einer -CH₂NH₂-Gruppe;
- einer -CH₂OH-Gruppe;
- einer Carboxylgruppe -COOH;
- einer Estergruppe -COOR₁,
wobei R₁ aus der aus Folgendem bestehenden Gruppe ausgewählt ist:
- einer linearen oder verzweigten Alkylgruppe mit einer Anzahl von Kohlenstoffatomen im Bereich von 1 bis 4;
- einer nichtsubstituierten Benzylgruppe;
- einer Polyethylenglykol-Methoxygruppe H(OCH₂CH₂)₁₋₄OH;
- einer Amidgruppe -CONR₂R₃,
wobei
- R₂ Wasserstoff ist und R₃ eine aus der aus Folgendem bestehenden Gruppe ausgewählte Gruppe ist:
- einer linearen oder verzweigten Alkylgruppe mit einer Anzahl von Kohlenstoffatomen, die im Bereich von 1 bis 4 umfasst ist;
- einer -(CH₂)₂NH₂-Gruppe;
- einer Phenylgruppe,
oder
- R₂ und R₃ lineare oder verzweigte Alkylgruppen mit einer Anzahl von Kohlenstoffatomen im Bereich von 1 bis 4 sind,
oder
- NR₂R₃ eine aus der aus Folgendem bestehenden Gruppe ausgewählte Gruppe ist:
- einem sekundären Cycloalkylamin mit einer Anzahl von Kohlenstoffatomen im Bereich von 4 bis 5;
- einem sekundären Heterocycloalkylamin, das ein unter Stickstoff und Sauerstoff ausgewähltes Heteroatom umfasst,
wobei die Koordinationsverbindung in flüssigkristalline lamellare und/oder nichtlamellare Lipidnanopartikel inkorporiert ist.

5. Arzneimittel für photodynamische Therapie nach Anspruch 4, wobei die Nanopartikel Cubosomen sind.

6. Arzneimittel für photodynamische Therapie nach Anspruch 4, wobei die Nanopartikel aus der aus Folgendem bestehenden Gruppe ausgewählt sind:
- Hexosomen;
- Ethosomen;
- Transferosomen;
- festen Lipidnanopartikeln;
- Nanoemulsionen;
- einer Kombination der Vorstehenden.

7. Arzneimittel für die photodynamische Therapie nach Anspruch 4, wobei die Nanopartikel Liposomen sind.

8. Verfahren zum Erlangen von Ruthenium(II)-Koordinationsverbindungen mit der folgenden Formel: 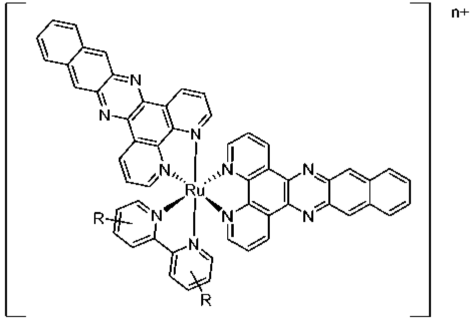
wobei n zwischen 0 und 2 ausgewählt ist,
wobei R eine Substituentengruppe ist, die aus der Gruppe ausgewählt ist, die aus Folgendem besteht:
- Wasserstoff;
- einer Methylgruppe;
- einer Phenylgruppe;
- einer -CH₂NH₂-Gruppe;
- einer -CH₂OH-Gruppe;
- einer Carboxylgruppe -COOH;
- einer Estergruppe -COOR₁,
wobei R₁ aus der aus Folgendem bestehenden Gruppe ausgewählt ist:
- einer linearen oder verzweigten Alkylgruppe mit einer Anzahl von Kohlenstoffatomen im Bereich von 1 bis 4;
- einer nichtsubstituierten Benzylgruppe;
- einer Polyethylenglykol-Methoxygruppe mit der Formel H(OCH₂CH₂)₁₋₄OH;
- einer Amidgruppe -CONR₂R₃,
wobei
- R₂ Wasserstoff ist und R₃ eine aus der aus Folgendem bestehenden Gruppe ausgewählte Gruppe ist:
- einer linearen oder verzweigten Alkylgruppe mit einer Anzahl von Kohlenstoffatomen im Bereich von 1 bis 4;
- einer -(CH₂)₂NH₂;
- einer Phenylgruppe,
oder
- R₂ und R₃ lineare oder verzweigte Alkylgruppen mit einer Anzahl von Kohlenstoffatomen im Bereich von 1 bis 4 sind,
oder
- NR₂R₃ eine aus der aus Folgendem bestehenden Gruppe ausgewählte Gruppe ist:
- einem sekundären Cycloalkylamin mit einer Anzahl von Kohlenstoffatomen im Bereich von 4 bis 5;
- einem sekundären Heterocycloalkylamin, das ein unter Stickstoff und Sauerstoff ausgewähltes Heteroatom umfasst,
wobei das Verfahren die Abfolge von folgenden Schritten umfasst:
- Erlangen eines reaktiven polymeren Vorläufers [Ru(CO)₂Cl₂] ₘ durch Reaktion zwischen Ruthenium(III)chlorid und Paraformaldehyd;
- Erlangen eines Zwischenkomplexes *trans*-Cl[Ru(NN)Cl₂(CO)₂]: 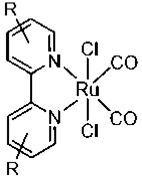
wobei NN ein 2,2'-Bipyridin-Ligand ist, der symmetrisch mit zwei gleichen funktionellen R-Gruppen funktionalisiert ist,
durch Reaktion des reaktiven polymeren Vorläufers [Ru(CO)₂Cl₂]ₘ mit einer vorbestimmten symmetrisch funktionalisierten 2,2'-Bipyridin-Gruppe mit den zwei gleichen funktionellen R-Gruppen,
- Erlangen der Ruthenium(II)-Koordinationsverbindungen durch Reaktion der Zwischenkomplexe 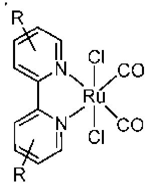
mit Benzo[i]dipyridin[3,2-a:2',3'-c]phenazin.

9. Verfahren nach Anspruch 8, wobei jede Substituentengruppe R an ein Kohlenstoffatom an einer Position gebunden ist, die ausgewählt ist unter der 5,5'-Position und der 4,4'-Position jedes Pyridinrings der 2,2'-Bipyridin-Gruppe

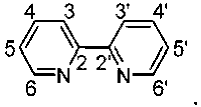

10. Verfahren nach Anspruch 8, wobei die Substituentengruppen R an Kohlenstoffatome an symmetrischen 5,5'- oder 4,4'-Positionen der jeweiligen Pyridinringe der 2,2'-Bipyridin-Gruppe 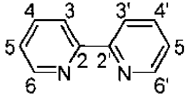 gebunden sind.

## Revendications

1. Composés de coordination de ruthénium (II) présentant la formule : 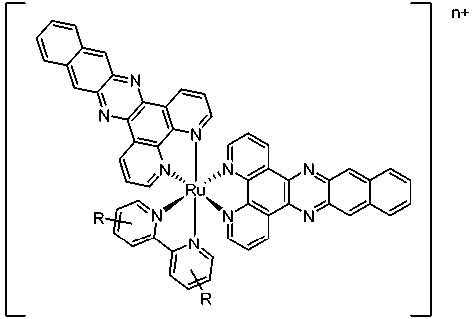
où n est choisi entre 0 et 2,
où R est un groupe substituant choisi dans le groupe constitué par :
- un groupe méthyle ;
- un groupe phényle ;
- un groupe -CH₂NH₂ ;
- un groupe -CH₂OH ;
- un groupe carboxyle -COOH ;
- un groupe ester -COOR₁, où R₁ est choisi dans le groupe constitué par :
- un groupe alkyle linéaire ou ramifié comportant un nombre d'atomes de carbone dans la plage de 1 à 4 ;
- un groupe benzyle non substitué ;
- un groupe méthoxy-polyéthylène glycol H(OCH₂CH₂)₁₋₄OH ;
- un groupe amide -CONR₂R₃, où
- R₂ est un hydrogène et R₃ est un groupe choisi dans le groupe constitué par :
- un groupe alkyle linéaire ou ramifié comportant un nombre d'atomes de carbone compris dans la plage de 1 à 4 ;
- un groupe -(CH₂)₂NH₂ ;
- un groupe phényle,
ou
- R₂ et R₃ sont des groupes alkyle linéaires ou ramifiés comportant un nombre d'atomes de carbone dans la plage de 1 à 4,
ou
- NR₂R₃ est un groupe choisi dans le groupe constitué par :
- une amine secondaire cycloalkyle comportant un nombre d'atomes de carbone dans la plage de 4 à 5 ;
- une amine secondaire hétérocycloalkyle comprenant un hétéroatome choisi parmi un azote et un oxygène,
destinés à être utilisés en tant qu'agents photosensibilisants dans une thérapie photodynamique pour le traitement de néoplasmes.

2. Composés de coordination destinés à être utilisés selon la revendication 1, dans lesquels chaque groupe substituant R est lié à un atome de carbone en une position choisie entre la position 5,5' et la position 4,4' de chaque cycle pyridine du groupe 2,2'-bipyridine :

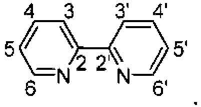

3. Composés de coordination destinés à être utilisés selon la revendication 1, dans lesquels lesdits groupes substituants R sont liés à des atomes de carbone en positions symétriques 5,5' ou 4,4' de cycles pyridine respectifs du groupe 2,2'-bipyridine

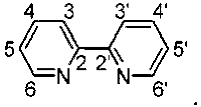

4. Médicament photosensibilisant destiné à une thérapie photodynamique pour le traitement de néoplasmes comprenant un composé de coordination de ruthénium (II) en tant qu'agent photosensibilisant, dans lequel ledit composé de coordination de ruthénium (II) présente la formule : 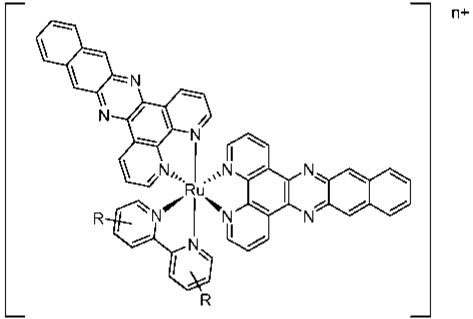
où n est choisi entre 0 et 2,
où R est un groupe substituant choisi dans le groupe constitué par :
- un hydrogène ;
- un groupe méthyle ;
- un groupe phényle ;
- un groupe -CH₂NH₂ ;
- un groupe -CH₂OH ;
- un groupe carboxyle -COOH ;
- un groupe ester -COOR₁,
où R₁ est choisi dans le groupe constitué par :
- un groupe alkyle linéaire ou ramifié comportant un nombre d'atomes de carbone dans la plage de 1 à 4 ;
- un groupe benzyle non substitué ;
- un groupe méthoxy-polyéthylène glycol H(OCH₂CH₂)₁₋₄OH ;
- un groupe amide -CONR₂R₃,
où
- R₂ est un hydrogène et R₃ est un groupe choisi dans le groupe constitué par :
- un groupe alkyle linéaire ou ramifié comportant un nombre d'atomes de carbone compris dans la plage de 1 à 4 ;
- un groupe -(CH₂)₂NH₂ ;
- un groupe phényle,
ou
- R₂ et R₃ sont des groupes alkyle linéaires ou ramifiés comportant un nombre d'atomes de carbone dans la plage de 1 à 4,
ou
- NR₂R₃ est un groupe choisi dans le groupe constitué par :
- une amine secondaire cycloalkyle comportant un nombre d'atomes de carbone dans la plage de 4 à 5 ;
- une amine secondaire hétérocycloalkyle comprenant un hétéroatome choisi parmi un azote et un oxygène,
dans lequel ledit composé de coordination est incorporé dans des nanoparticules lipidiques lamellaires et/ou non lamellaires liquides-cristallines.

5. Médicament destiné à une thérapie photodynamique selon la revendication 4, dans lequel lesdites nanoparticules sont des cubosomes.

6. Médicament destiné à une thérapie photodynamique selon la revendication 4, dans lequel lesdites nanoparticules sont choisies dans le groupe constitué par :
- des hexosomes ;
- des éthosomes ;
- des transférosomes ;
- des nanoparticules lipidiques solides ;
- des nanoémulsions ;
- une combinaison de ce qui précède.

7. Médicament destiné à une thérapie photodynamique selon la revendication 4, dans lequel lesdites nanoparticules sont des liposomes.

8. Processus permettant l'obtention de composés de coordination de ruthénium (II) présentant la formule : 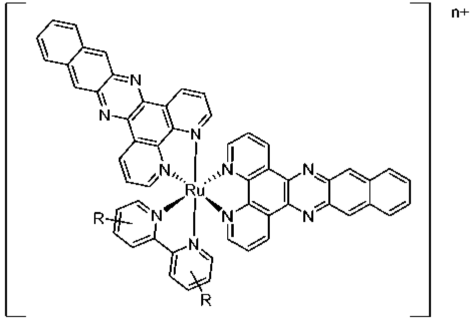
où n est choisi entre 0 et 2,
où R est un groupe substituant choisi dans le groupe constitué par :
- un hydrogène ;
- un groupe méthyle ;
- un groupe phényle ;
- un groupe -CH₂NH₂ ;
- un groupe -CH₂OH ;
- un groupe carboxyle -COOH ;
- un groupe ester -COOR₁,
où R₁ est choisi dans le groupe constitué par :
- un groupe alkyle linéaire ou ramifié comportant un nombre d'atomes de carbone dans la plage de 1 à 4 ;
- un groupe benzyle non substitué ;
- un groupe méthoxy-polyéthylène glycol de formule H(OCH₂CH₂)₁₋₄OH ;
- un groupe amide -CONR₂R₃,
où
- R₂ est un hydrogène et R₃ est un groupe choisi dans le groupe constitué par :
- un groupe alkyle linéaire ou ramifié comportant un nombre d'atomes de carbone dans la plage de 1 à 4 ;
- -(CH₂)₂NH₂ ;
- un groupe phényle,
ou
- R₂ et R₃ sont des groupes alkyle linéaires ou ramifiés comportant un nombre d'atomes de carbone dans la plage de 1 à 4,
ou
- NR₂R₃ est un groupe choisi dans le groupe constitué par :
- une amine secondaire cycloalkyle comportant un nombre d'atomes de carbone dans la plage de 4 à 5 ;
- une amine secondaire hétérocycloalkyle comprenant un hétéroatome choisi parmi un azote et un oxygène,
ledit processus comprenant la séquence d'étapes de :
- obtention d'un précurseur polymère réactif [Ru(CO)₂Cl₂]ₘ par réaction entre le chlorure de ruthénium (III) et le paraformaldéhyde ;
- obtention d'un complexe intermédiaire *trans*-Cl[Ru(NN)Cl₂(CO)₂] : 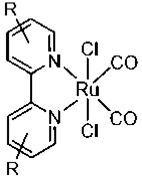
où NN est un ligand 2,2'-bipyridine fonctionnalisé de manière symétrique avec deux groupes R fonctionnels identiques,
par réaction dudit précurseur polymère réactif [Ru(CO)₂Cl₂]ₘ avec un groupe 2,2'-bipyridine prédéterminé fonctionnalisé de manière symétrique avec lesdits deux groupes fonctionnels R identiques,
- obtention desdits composés de coordination de ruthénium (II) par réaction desdits complexes intermédiaires 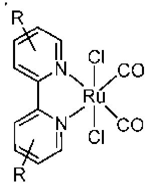
avec la benzo[i]dipyridine[3,2-a:2',3'-c]phénazine.

9. Processus selon la revendication 8, dans lequel chaque groupe substituant R est lié à un atome de carbone en une position choisie entre la position 5,5' et la position 4,4' de chaque cycle pyridine du groupe 2,2'-bipyridine

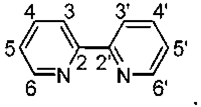

10. Processus selon la revendication 8, dans lequel lesdits groupes substituants R sont liés à des atomes de carbone en positions symétriques 5,5' ou 4,4' des cycles pyridine respectifs du groupe 2,2'-bipyridine
